# EUROPEAN PATENT SPECIFICATION

(11) **EP 4 157 215 B1**
(45) Date of publication and mention of the grant of the patent: **30.07.2025**
(21) Application number: 21730469.0
(22) Date of filing: 01.06.2021
(51) Int. Cl.: A61K 9/00, A61K 9/107, A61K 31/05, A61K 31/352, A61K 47/10, A61K 47/12, A61K 47/14, A61K 47/22, A61K 47/24

(54) **METHOD FOR SOLUBILIZING NATURAL, ENDOGENOUS AND SYNTHETIC CANNABINOIDS**
VERFAHREN ZUR SOLUBILISIERUNG VON NATÜRLICHEN, ENDOGENEN UND SYNTHETISCHEN CANNABINOIDEN
PROCÉDÉ DE SOLUBILISATION DE CANNABINOÏDES NATURELS, ENDOGÈNES ET SYNTHÉTIQUES

(30) Priority: 02.06.2020 EP 20000201
(43) Date of publication of application: 05.04.2023
(73) Proprietor: Athenion AG, 6304 Zug (CH)
(72) Inventor: SAAR, Ingo, 53589 Niederkassel (DE); BRYSCH, Wolfgang, 13505 Berlin (DE)
(74) Representative: Gruber, Daniel
(86) International application number: PCT/EP2021/000068
(87) International publication number: WO 2021/244771

(56) References cited:
- CA-A1- 3 050 013
- IZGELOV DVORA ET AL: "The effect of medium chain and long chain triglycerides incorporated in self-nano emulsifying drug delivery systems on oral absorption of cannabinoids in rats", INTERNATIONAL JOURNAL OF PHARMACEUTICS, ELSEVIER, NL, vol. 580, 6 March 2020 (2020-03-06), XP086127085, ISSN: 0378-5173, [retrieved on 20200306], DOI: 10.1016/J.IJPHARM.2020.119201
- "Phosal(TM) 53 MCT", INTERNET CITATION, 1 February 2007 (2007-02-01), pages 1, XP002601344, Retrieved from the Internet <URL:http://www.americanlecithin.com/TDS/TDS_53MCT.PDF> [retrieved on 20100917]

## Description

The present invention relates to a method for solubilizing phytogenic, endogenous and synthetic cannabinoids, to the solubilisate produced by this method and respective uses as a pharmaceutical dosage form.

Cannabinoids are compounds that act on cannabinoid receptors CB₁ and/or CB₂. There is a variety of naturally occurring cannabinoids of herbal origin (phytocannabinoids, mainly from *Cannabis sativa* and *Cannabis indica*)*,* endocannabinoids as endogenous cannabinoid receptor agonists and synthetic cannabinoids designed for pharmacological purposes.

Cannabis is the most widely used illicit drug worldwide that is consumed because of its psychotropic actions. But there is growing evidence that cannabinoids, the principal active agents in *Cannabis spec.,* display also beneficial effects on a plethora of disorders, if used properly. Therefore, there is a medical need to develop safe and targeted medications with a cannabinoid as pharmaceutically active agent. If used in low concentrations, some non-psychotropic cannabinoids can be also legally used in many countries as a dietary supplement.

The development of such medications, however, has been seriously limited by the poor bioavailability of the cannabinoids that can be achieved by oral application forms due to the highly lipophilic character of cannabinoids. In order to reach a sufficient bioavailability for psychotropic or pharmaceutical purposes cannabis is traditionally consumed in form of smoke of resins, cannabis oil or cannabis butter. These application forms, however, do not allow for administering a reliable dosage, as the concentrations of the active ingredients in the smoke or the cannabis oil are highly variable, as is the respective amount consumed by an individual user. Therefore, the absorbed concentrations can hardly be controlled. This renders these application forms impracticable and not manageable for medical treatment. Therefore, there is a medical need for a controllable pharmaceutical dosage form for cannabinoids that ideally is also consumer-friendly. A peroral dosage form that allows for an improved bioavailability is desirable. For selected cannabinoids an oral application form will also open the market for dietary supplements.

Previous attempts with oral dosage forms of cannabinoids (mainly tetrahydrocannabinol, THC) have met mixed success. Synthetic (-)-*trans*-Δ⁹-tetrahydrocannabinol (dronabinol) is marketed under the brand name Marinol^{®} for the treatment of HIV infection-induced cachexia (loss of weight and appetite), and of nausea and vomiting during cancer chemotherapy. In Marinol^{®} Δ⁹-tetrahydrocannabinol is dissolved in sesame oil and encapsulated in gelatin capsules. In comparison to application by smoke, only 25 - 30% of blood serum levels are achieved by peroral Marinol^{®} application and intestinal absorption. Maximal plasma levels are 10 ng/ml when administering 20 mg Δ⁹-tetrahydrocannabinol. While the onset of the psychotropic and beneficial effects of inhaled THC is almost immediately there is a delay of 0.5 to 2 hours with a peak effect after 2 to 4 hours when Marinol^{®} is administered perorally. There is a marked hepatic first pass effect so that only 10% to 20% of the administered dose will become systemically available. Because of the highly lipophilic nature of THC there is an accumulation in fatty tissue with an apparent volume of distribution of 10 L/kg leading to a prolonged excretion time. There is also a high plasma protein binding of ca. 97%. Moreover, there is a great patient interpatient variability (cf. Marinol - FDA prescription information side effects and uses; http://www.pdr.net/drug-summary/Marinol-dronabinol-2726, as of 20-05-2021).Therefore Marinol^{®} is a drug with a low bioavailability which is difficult to dose. These features have hampered so far a broader use.

Nabilone ((±)-trans-3-(1,1-dimethylheptyl)-6,6a,7,8,10,10a-hexahydro-1-hydroxy-6,6-dimethyl-9H-dibenzo[b,d]pyran-9-one) is a synthetic derivative of Δ⁹-tetrahydrocannabinol and has a similar use spectrum as dronabinol. At the moment it can be prescribed only in Canada and the UK for the treatment of loss of appetite and cachexia in AIDS patients.

Sativex^{®} (nabiximols) is a cannabinoid-based second line medication in form of an oral spray for the treatment of spasticity, neuropathic pain, overactive bladder in multiple sclerosis patients. Herein cannabis extracts are used that contain THC and cannabidiol roughly in a 1:1 ratio. Large variations in the effectiveness were found.

Further delivery systems for Δ⁹-tetrahydrocannabinol known in the art comprise a metered dose inhaler using non-CFC propellants (US 6,509,005; US 6,713,048), a pump action spray (US 6,946,150), a microsphere nasal delivery system (US 6,383,513), water-soluble prodrugs for intranasal administration (US 6,380,175), topical linement (US 6,949,582), cyclodextrin complexes (US 2005/0153931), solid lipid compositions ((US 5,891,469; US 5,989,583) and a targeted chylomicron/lipoprotein delivery (US 2014/0357708).

The first cannabidiol medication (Epidiolex^{®}) was approved in the USA in 2018 and in the European Union in 2019 for the treatment of two orphan childhood epilepsies, Dravet syndrome and Lennox-Gastaut syndrome.

In pharmacology, bioavailability is a parameter that indicates the fraction of an administered dose of unchanged drug that finally becomes available in the systemic circulation for the desired pharmacological effects. Poor bioavailability is often due to a poor water solubility, respectively the lipophilic nature of the active agent to be administered. Hence, the use of such substances as a dietary supplement or as a pharmaceutically active agent is impaired when using standard dosage forms.

There is a variety of approaches for improving the solubility of such agents and in many cases also their bioavailability by using solubilization techniques. Herein the solubility of an agent in a medium is augmented by adding a third substance. These third substances are referred to as solubilizers (solubilizing agents), substances that may for example build a complex with the substance to be solubilized. Examples for such chelating agents are sodium benzoate and sodium salicylate. Another mechanism of action of solubilizers is the augmentation of the dissolving capacity of the solvent, for example by disturbing the cluster structure of water. Examples for such structure breakers are glycerol (glycerin) and macrogols (polyethylene glycol, PEG).

US 6,946,150 B2 reveals a liquid formulation of a cannabinoid, alone or in combination with an opiate, to be used as a spray for inhalation, wherein the total amount of a solvent and a co-solvent is greater than 55% and no self-emulsifying agent is used. The mean aerodynamic particle size is 20 -40 microns.

A third solubilization mechanism are micelle and liposome application technologies. They have won broad attention throughout the last decades. Herein the substance to be delivered is enclosed in a spherical aggregate of surfactant molecules. These molecules are characterized by a polar head group and a long nonpolar chain ("tail"). When given into an aqueous medium these molecules tend to associate by aggregating to spherical structures by orienting the polar head group towards the surrounding medium and the nonpolar chain towards the interior of the spheres. When these spheres consist of only one layer of such amphiphilic molecules they are referred to as micelles. Depending on the nature of the amphiphilic molecule and the reaction conditions it is also possible to form spheres with more than one layer. Herein a second layer is formed inside the outer layer of the sphere, the nonpolar groups of this second layer being oriented towards the nonpolar groups of the outer layer, and the polar head groups being oriented towards the interior of the sphere. Such aggregates are referred to as liposomes. In their structure they resemble the lipid bilayer of the cell membrane. There are also multi-layered liposomes in which at least two liposomal spheres are formed concentrically around one another, thus building a multispherical aggregate. When given in a lipophilic medium these substances tend to build inversed spherical structures where the lipophilic chain is oriented towards the solution medium and the other layers are arranged accordingly.

Different uses of such loaded spheres have been described in the art, among them the usage as a dosage form for the application of lipophilic substances and/or for increasing the bioavailability of the enclosed substance. In micelles, the enclosed nonpolar substance concentrates in the interior space of the sphere toward which the nonpolar chains of the amphiphilic molecules are oriented. In liposomes, however, the interior space of the spheres is an aqueous, respectively hydrophilic medium. It can serve for packaging hydrophilic molecules. Poorly water-soluble, respectively lipophilic molecules, however, gather mostly in between the lipophilic structures of the liposomal layers.

The use of styrene maleic acid nanomicelles for encapsulating the synthetic cannabinoid WIN55,212-2 is disclosed by Xian et al., 2015, Anticancer Res. 35, p. 4707-4712. A formulation comprising a unilamellar micelle or liposome suspension of a cannabinoid that is thermostable at temperatures above 50°C is disclosed in US 2016/0030387 A1.

A micelle-based solubilisate for solubilizing dietary supplements is known for ubiquinone Q₁₀ (WO 03/007907 A1) or curcumin (WO2014/094921 A1). Therein an emulsifier with a HLB (hydrophilic-lipophilic balance) value of 9 - 16 or 13 - 18 is used, respectively. Polysorbate (Tween) 20 or 80 is preferred. From empiric pharmacokinetic measurements it is known that the organism can absorb micelles as well as liposomes in the gastrointestinal tract via the intestinal villi. However, their degree of absorption seems to be rather variable and therefore these methods have met a mixed success for augmenting the bioavailability of the enclosed compound. The transport, respectively the absorption rate over the cell membrane is an intrinsic characteristic for each substance, depending on a variety of factors such as molecule size, degree of lipophilicity and the presence of suitable transporter molecules inside the cell membrane. For many compounds these parameters are not known and would have to be determined first before finding a suitable packaging for a specific compound.

Liposomal applications have been widely discussed in medicine and pharmacology and some sophisticated solutions have been developed for specific active agents. Their use, however, is not very common. One reason are the relatively high production costs, another reason are possible adverse side effects. In particular, when parenterally applied, liposomes carry the risk of accumulating in the liver, the spleen and/or the bone marrow. Therefore, liposomal formulations are often viewed skeptically.

A nano-liposphere-based formulation method for increasing drug bioavailability was disclosed in WO 2013/108254. Although this method offers some advancement over the state-of-the-art there are also some inherent drawbacks. High-pressure homogenizers are needed for the production of these solid lipid nanoparticles. However, high-pressure induced drug degradation has been described for some drugs or dietary supplements. Lipid crystallization, gelation phenomena and co-existence of several colloidal species occur. Further restrictive factors such as cytotoxic effects after phagocytosis, toxic effects of organic residues and a difficult industrial scale-up have limited their use until now (Mehnert and Mäder, Adv Drug Deliv Res 2001, 47, 165-196; Dudala et al., Int J Pharm Investg 2014, 4, 149-155). Moreover, their drug loading capacity is relatively small and they display a low viscosity. This makes them not very attractive for topic or transdermal application forms (Mukherjee et al., Indian J Pharm Sci 2009, 71, 349-358). Further, the use of an amphiphilic solvent such as lower alkyl esters of lactic acid or N-methylpyrrolidone is required in WO 2013/108254. N-methylpyrrolidone is listed as a substance of very high concern in respect of being potentially carcinogenic and toxic for reproduction, methyl lactate is usually hydrolyzed to lactate and methanol in an aqueous environment. Ethyl lactate etc. is well tolerated. However, due to relatively high production costs it is not a very attractive solvent, particularly not for dietary supplements.

Another solubilization technique is the formation of inclusion complexes of the substance to be solubilized with cyclodextrins such as α-, β- or γ-cyclodextrin or cyclodextrin derivatives such as 2-hydroxypropyl-β-cyclodextrin, methyl-β-cyclodextrin or trimethyl-β-cyclodextrin. Typically, cyclodextrins are composed of 6 to 8 1,4-linked α-D-glucopyranosides forming macrocycles. Thus a water-soluble toroid (cone-shaped or bucket-shaped) structure is generated which is capable to host hydrophobic substances in its interior. The interior space is considerably less hydrophilic than the outside contacting the aqueous environment. Cyclodextrins are produced from starch by enzymatic treatment. They are loaded with the compound to be solubilized by dispersion. The compound to be solubilized can then be released by contacting these complexes with water, by pH or temperature changes, depending on the specific composition. However, the development of cyclodextrin is apparently not easy and relatively costly. This limited their use until now. A cyclodextrin-based formulation method for cannabinoids is disclosed in WO 03/070774 A1.

CA 3050013 A1 reveals lecithin-based compositions for solubilizing cannabinoids using i.a., polyethylene glycols and/or polysorbates that evoke health concerns. Some examples use a prefabricated mixture Phosal 53 MCT that cannot be adapted easily.

Izgelov et al. (2020, Int J Pharm 580: 119201 describes lecithin-based compositions of cannabinoids and their optimization with regard to particle size. All these formulations recur to polyethylene glycols and/or polysorbates.

Thus, all these techniques have their advantages but also some drawbacks.

As can be seen, there is a variety of methods in the art how to solve cannabinoids. A major problem of many of these methods is that they need therefor specific emulsifiers. As cannabinoids such as Δ⁹-tetrahydrocannabinol or (-)-cannabidiol are intended for a chronic use, for example in pain therapy, it is of uttermost importance that these emulsifiers not only are pharmaceutically acceptable, but they should also be permitted for food use. Some adverse side effects of emulsifiers might be seen as acceptable in a short-term medication but should be as far as possible avoided in a long-term, respectively chronic medication. Two groups of such problematic emulsifiers are polysorbates (Tween) and polyethylene glycols (PEGs).

In experimental studies cannabinoids were solubilized by means of polysorbates such as Tween 80 (e.g. Coutts and Pertwee, 1997, Br J Pharmacol, 121, 1557-1566; Goonawardana et al., 2011, Hippocampus, 21, 520-531). Polysorbates ((Tween^{®} 20, 40, 60, 65 or 80) are widely used in solubilization techniques. While polysorbates are valuable solubilizers in experimental settings they are less suitable for pharmacological or nutraceutical purposes because of health concerns. Until now, the implementation of this technology is used in the production of chewing gum, as emulsifier in the food industry and for some parenteral pharmaceutical formulations. However, there is an ongoing controversy about a detrimental impact of polysorbates on health. Polysorbate 20 was found to be contaminated with unreacted 1,4-dioxane and ethylene oxide. Both are known to be carcinogenic compounds (cf. FDA 1999, 21 CFR Part 173, Federal Register Vol 64, No. 104, pp. 29224-29227). Polysorbates such as Tween 80 are known to reduce the efficacy of preservatives such as parabens by binding to them (cf. Blanchard et al. (1977) J Pharm Sci 66: 1470-1473). The paraben concentration, however, should not be increased accordingly because of their estrogenic potential (cf. Okubo et al. (2001) Food Chem Toxicol 39: 1225-1232). Hypersensitivity reactions to polysorbates (in particular polysorbate 80) are common adverse side effects (cf. Steele et al. (2005) Nephrology 10: 317-320; Norris et al. (2010) Commun Oncol 7: 425-428).

A major problem, however, seems to be the detrimental effect of Tween 80 on the gut microflora. Apparently, it favors the development of obesity and inflammatory bowel diseases such as colitis ulcerosa and Crohn's disease (Roberts et al. (2013) J Crohns Colitis 7: 338-341; Chassaing et al. (2015) Nature 519: 92-96). This is in line with a finding from a clinical study with a turmeric medicament containing polysorbate 80 as emulsifier. There was a dropout rate from the study of about 50%. The major adverse side effect was diarrhea which can't be contributed to turmeric (cf. Henrotin et al. (2019) Arthritis Res Ther21: 179).

Polysorbate 80 absorption leads to a sustained decrease in blood pressure due to negative inotropic effects (Gough et al. (1982) J Cardiovasc Pharmacol 4: 375-380). It has been associated with systemic hypotension in amiodarone formulations where this may even lead to casualties (cf. Cushing et al. (2009) Eur J Pharmacol 607: 167-172).

Polysorbate 80 alters membrane fluidity and increases membrane permeability (Anderberg et al. (1992) J Pharm Sci 81: 879-887). Polysorbate 80 increases the susceptibility of cells to oxidative stress at clinically relevant concentrations (Tatsuishi et al. (2005) Toxicol 207: 7-14). Cytotoxic effects of polysorbate 80 are attributed to the formation of peroxides that interfere with cell proliferation (cf. Kubis et al. (1979) Pharmazie 34: 745-746; Chajes et al. (1995) Breast Cancer Res Treat 34: 199-212). Polysorbate 80 has been implicated in cases of renal and liver toxicity (Rhodes et al. (1993) Gut 34: 565-566; Curran et al. (2002) Crit Care Resusc 4: 112-115; Bove et al. (1985) JAMA 254: 2422-2430). A study raised concerns that polysorbate-80 might decrease fertility in rats (Gajdova et al. (1993) Food Chem Toxicol 31: 183-190). A further problem of the use of polysorbate (Tween)-based solutions is their soapy taste. This renders them unattractive for dietary supplements and liquid oral medications.

The addition of polyethylene glycols (PEGs, macrogols) as an emulsifier, respectively the so-called PEGylation by linking PEGs to poorly water-soluble active agents are well-established methods in pharmaceutics. While the solubilization is usually good there is increasing evidence that PEGs might cause health hazards in long-term administration. This renders their use undesirable at least in food and cosmetics. Their use should therefore be preferentially limited to short-term medications.

Recently, physicians became aware of the allergic potential of PEGs that may even lead to anaphylaxis (Wylon et al. (2016) Allergy Asthma Clin Immunol 12: 67; Sellaturay et al. (2021) J Allergy Clin Immunol Pract 9: 670-675; Brousgaard-Mouritsen et al. (2021) Clin Exp Allergy 51: 463-470). Anti-PEG antibodies can be formed. Because of the ubiquitarian PEG products even persons who never took a PEG-containing medication may have anti-PEG antibodies (Thi (2020) Polymers 12: 298). In particular PEGylated micelle carrier systems such as PEG-PBLA micelles are suspected to cause immunogenicity issues (Shiraishi and Yokoyama (2019) Science and Technology of Advanced Materials 20: 324-336).

The non-biodegradability of PEG limits the capacity of the kidneys to clear it. In consequence, there is an accumulation in the liver and in lysosomes of normal tissues. This can lead to the so-called macromolecular syndrome. The PEGylation-induced enlargement of nanoparticles may increase the reticuloendothelial system uptake, resulting in an unfavorable fast release of drug cargoes (Thi (2020) Polymers 12: 298).

In rats, the gavage of PEG 400 led to gastric mucosal changes with an infiltration of eosinophils and globule leukocytes (Ueda et al. (2011) J Toxicol Sci 36: 811-815).

In zebrafish, PEGylation of silicon nanoparticle amino groups increased their toxicity (Calienni et al. (2019) Nanomedicine 14: 375-385). The addition of PEG increased the toxicity of imidacloprid in Japanese quails (Rawi et al. (2019) Metab Brain Dis 34: 443-450).

For example, PEG-3350 is an osmotic laxative that retains water in the stool. It can, however, cause side effects such as diarrhea, nausea, vomiting, stomach cramps, irritation of the rectum, excessive thirst, sleep disorders, abdominal pain and bloating (cf. MedlinePlus, National Institute of Health; WebMD, Polyethylene Glycol 3350; as of May 13^{th}, 2021).

According to the European Codex alimentarius, PEG (= E1512) is allowed in water-based drinks in an amount of 1 mg/kg (FAO and WHO, 2021). When PEG would be used as an emulsifier this limit would be passed.

Kolliphor^{®} RH 40 (previously Cremophor^{®} RH 40) is the trade name of PEG 40 hydrogenated castor oil (macrogolglycerol hydroxystearate). It is widely used in SEDDS (self-emulsifying drug delivery systems) and SMDDS (microemuslifying systems). Cremophor^{®} RH 40 in concentrations corresponding to clinical dosages was found to cause endothelial and epithelial toxicity, as was verified by lactate dehydrogenase release and in 3-(4,5-dimethylthiazol-2-yl)-2,5-diphenyltetrazolium bromide (MTT) assays (Kiss et al. (2013) J Pharm Sci 102: 1173-1181).

There is a thus need to find a solubilization method for solubilizing poorly water-soluble cannabinoids that does not need polysorbates, polyethylene glycols or the like.

It should fulfil the following criteria:
- easy-to-handle
- no lengthy development time for finding a favorable composition
- no costly equipment needed
- inexpensive materials and production costs
- applicable for a broad range of poorly water-soluble cannabinoids
- no addition of polysorbates /or polyethylene glycols needed

Particularly for dietary supplements the development and production costs are a serious obstacle for developing suitable solubilization techniques, as the obtainable price on the market is limited.

Surprisingly, it was found that the method according to the disclosure is able to solve this task.

Herein, at least one cannabinoid is solubilized by the method according to the invention, comprising the following steps:
a) Providing a first composition comprising at least one cannabinoid in the overall range of 10 % to 45 % per weight of the first composition and at least one medium-chained triglyceride in the overall range of 55 % to 90 % per weight of the first composition, wherein said at least one cannabinoid and said at least one medium-chained triglyceride are mixed under stirring in a temperature range of 15°C to 40°C and the relative weight percentages of the first composition add up to 100%;
b) Providing a second composition comprising at least one non-hydrogenated phosphatidylcholine in the overall range of 60 % to 85 % per weight of the second composition, at least one non-hydrogenated lysophosphatidylcholine in the overall range of 5 % to 30 % per weight of the second composition, at least one C₂-C₄ alcohol in the overall range of 3 % to 30 % per weight of the second composition and at least one of glyceryl stearate or a saturated or unsaturated C₁₄ to C₂₀ fatty acid in the overall range of 1 % to 15 % per weight of the second composition, wherein said ingredients are mixed under stirring at room temperature, the weight ratio of said at least one non-hydrogenated phosphatidylcholine to said one non-hydrogenated lysophosphatidylcholine is in the range of 15:1 to 2:1 and the relative weight percentages of the second composition add up to 100%;
c) Mixing said first composition and said second composition under stirring and the use of ultrasonic treatment in a frequency range of 20 kHz to 1 MHz and a power range of 50 W to 40 KW,
   wherein the weight ratio of said first composition to said second composition is in the range of 9:1 to 3:2;
d) Heating the mixture of step c) by continuously increasing the temperature with a temperature increment of 0.5°C/min to 5°C/min until a final temperature of 100°C
   to 130°C is reached,
e) Optionally maintaining the final temperature of step d) for a time range of 1 hour to 2 hours; and
f) Letting the resulting solubilisate cool down to room temperature,
wherein no polysorbate or polyethylene glycol is used for the solubilization process.

A major advantage of this method of solubilization is that the cannabinoids according to the disclosure can be solubilized without the help of hazardous emulsifiers such as polysorbates or polyethylene glycols. At the contrary, phosphatidylcholines are said to be cell-protective (cf. for example Ehehalt et al. (2010) Biochim Biophys Acta 1801: 983-993; Tokés et al. (2011) Shock 36: 458-465; Zhang et al. (2014) J Membr Biol 247: 73-80).

It could be shown in Experiments 1 (Δ⁹-tetrahydrocannabinol), 2 ((-)-cannabidiol) and 3 (*Cannabis sativa* extract) that the method of the disclosure yields an excellent solubilization. The resulting solubilisate is apt to be dissolved in an aqueous solution for providing a finished solution.

Confusing and even contradictory definitions can be found in the art. In order to avoid any ambiguity a solubilisate according to the invention is defined as follows:
A **solubilisate** is the composition of the at least one substance to be solubilized and the solubilizing agents as defined according to the invention. Further addition of a solvent or diluent shall not be covered by this term. A solvent or diluent is not included in a solubilisate according to the invention. The solubilisate according to the invention is produced first by a solubilization method according to the invention, then a specific nutritional or pharmaceutical composition is produced with said solubilisate, and finally said nutritional or pharmaceutical composition is packaged into a suitable container for the respective product.

It is characterized by the substantially complete solubilization of the at least one cannabinoid, thus being a nearly perfect solution in which the molecules behave completely as independent entities in a solution and substantially undergo the distribution and thermodynamic rules of Brownian motion. Thus the solubilisate is a clear solution containing the respective cannabinoid in a high concentration. In general, the solubilisate is not meant for intake without dilution. In most cases, a portioned solubilisate accounts to a volume of a few milliliters.

In the scope of this patent application the terms **"solubilization aggregate"** or **"solubilization essence"** shall be used synonymously to "solubilisate".

The term **"pre-solubilized"** refers to an intermediate stage in the method of the invention at which the mixed ingredients display already a solubilized appearance, but however either don't show long-term stability yet, show a increased tendency to partially crystallize, an increased tendency to phase separation after cooling down, or to less vesicle formation after adding the solubilisate to an aqueous solution.

A solubilisate according to the invention must be differentiated from a **suspension** (colloidal suspension). This term defines a heterogeneous mixture containing solid particles that sooner or later will undergo sedimentation. It is also different from an **emulsion** (a mixture of two liquids which usually are immiscible). For increasing the bioavailability and/or resorption of a substance the complete solubilization is highly preferably. Therefore, solubilisates are preferred over suspensions or emulsions.

A solubilisate according to the invention must also be differentiated from a **concentrate.** A concentrate is a compound, respectively a composition of compounds without a diluent. Upon release of a concentrate into a diluent the concentrate dissolves itself either completely in the diluent or forms a suspension or emulsion with the diluent. A concentrate does not need the interaction with solubilizing agents, as it is intrinsically solvable in water or an aqueous solution.

A solubilisate according to the invention must also be differentiated from a **lyophilizate** that has been reconstituted in any form of solvent. Freeze-drying is a dehydration technique for preserving perishable compounds, preferentially pharmaceutically active agents. A formulation of the at least one compound is frozen and then the surrounding pressure is reduced so that the frozen water in the material is sublimed directly from the solid phase to the gas phase. When properly sealed, the resulting lyophilizate can be stored at room temperature. Thus, the shelf life of a sensitive product can be significantly increased. The product can be reconstituted by dissolving the lyophilizate in a suitable solvent. Often a lyoprotectant or cryoprotectant is added.

The term solubilisate used according to the invention must be differentiated from the **finished solution,** respectively the prepared beverage or fluid oral dosage form to be imbibed. This finished solution according to the invention is generated by diluting the solubilisate according to the invention in a diluent, preferably an aqueous solution, in order to produce a beverage, respectively any fluid oral dosage form ready for intake by the consumer, respectively the patient.

A **diluent** in the scope of the present application is a diluting agent (dilutant, thinner). It is not part of the solubilisate according to the invention.

In the scope of the present application the term **"solubilizing agent"** refers to any chemical substance that is added to the cannabinoid for solubilizing it so that the cannabinoid can be solved thereupon in an aqueous solution. The term **"solubilizer"** shall be used synonymously.

In the scope of the present application the term "medicine" shall comprise human and veterinary medicine.

A great advantage of such a solubilisate consists in its small volume. Thus, it can be easily portioned to patient- or consumer-friendly units, or relatively huge amounts of a solubilized substance can be shipped at low costs. For producing a finished solution, the dilution of the solubilisate in an aqueous medium (e.g. tap water or mineral water) can be easily carried out by medical staff, patients, or consumers.

In a preferred embodiment of the method according to the invention the at least one cannabinoid is provided in the overall range of 2 % to 15 % per weight, in a more preferred embodiment in the overall range of 2 % to 10 % per weight.

Known phytocannabinoids from *Cannabis spec.* comprise cannabinoids of the
- tetrahydrocannabinol type (THC) such as Δ⁹-tetrahydrocannabinol (Δ⁹-THC-C₅), Δ⁹-tetrahydrocannabinol C₄ (Δ⁹-THC-C₄), (-)-Δ⁸-trans-(6aR,10aR)-Δ⁸-tetrahydrocannabinol (Δ⁸-THC-C₅), Δ⁹-tetrahydrocannabinolic acid A (Δ⁹-THCA-C₅A), Δ⁹-tetrahydrocannabinolic acid B (Δ⁹-THCA-C₅ B), Δ⁹-tetrahydrocannabinolic acid C₄ A (Δ⁹-THCA-C₄A), Δ⁹-tetrahydrocannabinolic acid C₄ B (Δ⁹-THCA-C₄ B), (-)-Δ⁸-trans-(6aR,10aR)-Δ⁸-tetrahydrocannabinolic acid A (Δ⁸-THCA-C₅ A), (-)-(6aS,10aR)-Δ⁹-tetrahydrocannabinol ((-)-cis-Δ⁹-THC-C₅), Δ⁹-tetrahydrocannabivarin (Δ⁹-THCV-C₃), Δ⁹-tetrahydrocannabivarinic acid A (Δ⁹-THCVA-C₃ A), Δ⁹-tetrahydrocannabiorcol (Δ⁹-THCO-C₁), Δ⁹-tetrahydrocannabiorcolic acid A (Δ⁹-THCOA-C₁ A), Δ⁹-tetrahydrocannabiorcolic acid B (Δ⁹-THCOA-C₁ B). THC is the most important psychoactive substance in *Cannabis spec..* It can amount for up to 22 wt.-% of the *Cannabis* extract. However, in most *Cannabis* plants it is around 10%. (-)-Δ⁹-trans-THC is 6 to 100 times more effective than (+)-Δ⁹-trans-THC. In contrast, cis isoforms have no psychotropic effects. THC acts as a partial agonist at the CB₁ and CB₂ receptors. Its binding at the CB₁ receptor induces a feeling of happiness, relaxation and analgesia. Common effects are mood enhancement, euphoria, loquacity, altered sensual perception and increased judiciousness. On the other hand, detrimental actions such as impaired cognitive abilities, impaired memory and concentration, disruptive thinking, ataxia, tremor, dizziness, feelings of unreality, depersonalization and detachment, panic, anxiety, dysphoria, paranoia, tachycardia, orthostatic hypotonia, irritation of conjunctiva, xerostomia, endocrine and reproductive impairment, altered thermoregulation and mydriasis are the most common adverse effects of THC. The actions at the CB₂ receptor have been associated with immunomodulation. THC is an antagonist of the 5-HT₃ receptor what accounts for its antiemetic effects. Therefore, it is used to alleviate nausea in patients undergoing a chemotherapy. THC is a positive allosteric modulator of µ- and δ-opioid receptors. The analgesic effects refer to an alleviation of neuropathic and inflammatory pain. Antioxidative properties have been described. Therefore, it is thought to be neuroprotective, e.g. in the prophylaxis and treatment of Parkinson's disease, Alzheimer's disease and Huntington's disease. In tumor cells it can promote apoptotic and necrotic cell death. Further. the spreading of tumor cells is inhibited. Spasticity is alleviated. Gastrointestinal motility is deaccelerated. The intraocular pressure is lowered. Sleep is facilitated. THC stimulates the appetite. Clinical studies are underway for testing the efficacy of THC in the treatment of glaucoma, multiple sclerosis, Crohn's disease, ulcerative colitis and Tourette syndrome.
- cannabidiol type (CBD) such as (-)-cannabidiol (CBD-C₅), cannabidiol-C₄ (CBD-C₄), Δ³-cannabidiol, Δ^{3,7}-cannabidiol, Δ²-cannabidiol, Δ⁶-cannabidiol, cannabidiolic acid (CBDA-C₅), cannabidiol monomethyl ether (CBDM-C₅), (-)-cannabidivarin (CBDV-C₃), cannabidivarinic acid (CBDVA-C₃), cannabidiorcol (CBD-C₁). CBD may make up to 40 wt.-% of *Cannabis* extracts. CBD shows apparently no psychotropic effects. It binds with a very low affinity to the CB₁ and CB₂ receptors, acting as an indirect antagonist at these receptors. Further, there are antagonistic actions at the G protein-coupled receptor GPR55. CBD is a partial agonist at the 5-HT_{1A} receptor. There may be an effect on the mitochondrial voltage-gated ion channel VDAC₁. Allosterically it modulates µ and δ opioid receptors. CBD is an active ingredient of nabiximols that it used for spasmolysis in multiple sclerosis patients. CBD is classified as an orphan drug for certain childhood epilepsies such as Dravet syndrome and Lennox-Gastaut syndrome (LGS). Immunosuppressive actions have been described. Therefore, beneficial effects are discussed for the therapy of inflammatory bowel diseases such as Crohn's disease and ulcerative colitis, acute lung injury and acute pancreatitis. Linked to that it may alleviate chronic inflammatory and neuropathic pain. Its antioxidative properties make it a candidate for the prophylaxis and treatment of neurogenerative diseases. The actions at the 5-HT_{1A} receptor may be responsible the antidepressant, anxiolytic and neuroprotective effects of CBD. Beneficial effects were reported in the management of drug addiction, e.g. of tobacco, cannabis, opioids and psychostimulants.
- cannabinol type (CBN) such as cannabinol (CBN-C₅), cannabinol-C₄ (CBN-C₄), cannabivarin (CBV; CBN-C₃), cannabinol-C₂ (CBN-C₂), cannabiorcol (CBN-C₁), cannabinolic acid A (CBNA-C₅A), cannabinol methyl ether (CBNM-C₅). It is generated through oxidation of THC. CBN is not psychoactive and binds with a low affinity to the CB₁ receptor as a partial agonist and with a higher affinity to the CB₂ receptor, but comparatively weaker than THC. CBN shows analgesic actions. Immunosuppressive and anti-inflammatory effects via the CB₂ receptor were described. Here it may trigger apoptosis and reduce cytokine production.
- cannabinodiol type (CBND, sometimes also CBDL) such as cannabinodiol (CBND-C₅) and cannabinodivarin (CBND-C₃). CBND is a completely aromatized derivative of CBD. It shows psychotropic effects and can occur after photochemical conversion of CBN.
- cannabigerol type (CBG) such as cannabigerol ((E)-CBG-C₅), cannabigerol monomethyl ether ((E)-CBGM-C₅ A), cannabigerolic acid A ((E)-CBGA-C₅ A), cannabigerolic acid A monomethyl ether ((E)-CBGAM-C₅ A), cannabigerovarin ((E)-CBGV-C₃), cannabinerolic acid A ((Z)-CBGA-C₅ A), cannabigerovarinic acid ((E)-CBGVA-C₃ A). CBG binds as an agonist with a high affinity to the α₂ receptor, moderately as an antagonist at the 5-HT_{1A} receptor and acts as a weak CB₁ and CB₂ receptor antagonist. It doesn't show psychotropic actions. It was found that CBG reduces the intraocular pressure in cats. This may be beneficial in the treatment of glaucoma. It has a potential for alleviating pain, anxiety, depression and it lowers muscle tension. In animals it was effective against inflammatory bowel diseases such as Crohn's disease and ulcerative colitis. It has antimicrobial activity against *Mycobacterium intracellulare* and possibly also MRSA (methicillin-resistant *Staphylococcus aureus*)*.* Neuroprotective effects have been described that may be useful a.o. in the treatment of multiple sclerosis and Huntington's Disease. There might be a potential in the treatment of a variety of tumors such as breast, liver, lung, pancreatic, skin, ovarian, renal, bladder and colon cancer. CBG seems to have skin moisturizing effects.
- cannabichromene type (CBC) such as (±)-cannabichromene (CBC-C₅), (±)-cannabichromenic acid A (CBCA-C₅ A), (±)-cannabivarichromene (= (±)-cannabichromevarin; CBCV-C₃), (±)-cannabichromevarinic acid A (CBCVA-C₃ A). CBC is classified as non-psychotropic. It interacts with the CB₁ and CB₂ receptors. Many of its actions are contributed to the actions at the TRPV1 and TRPA1 receptors. CBC *per se* has no analgesic effects but it supports the pain-relieving actions of THC. Tranquilizing actions have been observed for CBC. It has a role in the antiviral and anti-inflammatory effects of *Cannabis* extracts. There may be also antifungal actions. CBC may be useful in dermatology for skin inflammations and acne treatment. Intestinal hypermotility is promoted without creating constipation. Antidepressant properties have been described. CBC activates antinociceptive pathways. It helps to promote neurogenesis.

Smaller groups of cannabinoids comprise the following groups which were isolated and chemically identified but their physiological effects are poorly understood or not investigated yet: Some of them are generated as metabolites of the aforementioned cannabinoids.
- cannabitriol type (CBT) such as (-)-(9R,10R)-trans-cannabitriol ((-)-*trans*-CBT-C₅), (+)-(9S,10S)-cannabitriol ((+)-*trans*-CBT-C₅), (±)-(9*R*,10*S*/9*S*, 10R)-cannabitriol ((±)-*cis*-CBT-C₅), (-)-(9*R*,10*R*)-*trans*-10-O-ethyl cannabitriol ((-)-trans-CBT-OEt-Cs), (±)-(9*R*,10*R*/9*S*, 10S)-cannabitriol C₃ ((±)-*trans*-CBT-C₃), 8,9-dihydroxy-Δ^{6a(10a)} tetrahydrocannabinol (8,9-di-OH-CBT-C₅), cannabidiolic acid A cannabitriol ester (CBDA-C₅ 9-OH-CBT-C₅ ester), (-)-(6aR,9S,10S,10aR)-9,10-dihydroxy hexahydrocannabinol (= cannabiripsol, cannabiripsol-C₅), (-)-6a,7,10a-trihydroxy Δ9-tetrahydrocannabinol ((-)-cannabitetrol), 10-oxo-Δ^{6a(10a)} tetrahydrocannabinol (OTHC).
- cannabielsoin type (CBE) such as (5a*S*,6*S*,9*R*,9a*R*)-cannabielsoin (CBE-C₅), (5a*S*,6*S*,9*R*,9a*R*)-C₅ cannabielsoin (CBE-C₃), (5a*S*,6*S*,9*R*,9a*R*)-cannabielsoic acid A (CBEA-C₅ A), (5a*S*,6*S*,9*R*,9a*R*)-cannabielsoic acid B (CBEA-C₅ B), (5a*S*,6*S*,9*R*,9a*R*)-C₃ cannabielsoic acid B (CBEA-C₃ B), cannabiglendol C₃ (OH-iso-HHCV-C₃), dehydrocannabifuran (DCBF-C₅), cannabifuran (CBF-C₅). CBE is a metabolite of CBD.
- cannabicyclol type (CBL) such as (±)-(1a*S*,3a*R*,8b*R*,8c*R*)-cannabicyclol (CBL-C₅), (±)-(1a*S*,3a*R*,8b*R*,8c*R*)-cannabicycloic acid A (CBLA-C₅ A), (±)-(1a*S*,3a*R*,8b*R*,8c*R*)-cannabicyclovarin (CBLV-C₃). CBL does not trigger psychoactive effects. CBL is a product of light oxidation of CBC.
- cannabicitran type (CBT) such as cannabicitran (CBT-C₅).
- cannabichromanone type (CBCN) such as cannabichromanone (CBCN-C₅), cannabichromanone C₃ (CBCN-C₃), cannabicoumaronone (CBCON-C₅). A strong antioxidant activity of these compounds has been observed.
- isocannabinoids such as (-)-Δ⁷-*trans*-(1*R*,3*R*,6*R*)-isotetrahydrocannabinol, (±)-Δ⁷-1,2-*cis*-(1*R*,3*R*,6*S*/1*S*,3*S*,6*R*)-isotetrahydrocannabivarin, (-)-Δ⁷-*trans*-(1*R*,3*R*,6*R*)-isotetrahydrocannabivarin.

Endocannabinoids are endogenous neurotransmitters of the endocannabinoid system. They comprise anandamide, 2-arachidonyl glycerol, O-arachidonyl ethanolamide, N-arachidonoyl dopamine, γ-linolenoyl ethanolamide, docosatetraenoyl ethanolamide and 2-arachidonyl glyceryl ether. Anandamide is pain-relieving (probably via its action at the TRPV1 receptor) and is under investigation for its effects on eating and sleep disorders. It has been shown to impair the working memory in rats. It may be also a biomarker for infertility. To the group of endocannabinoids belongs also N-palmitoyl ethanolamine (PEA) which provides an antioxidative protection against UV-B in the skin. Several medical uses are discussed for some of these substances, however hitherto with mixed success.

Synthetic cannabinoids have been synthesized for replacing banned phytocannabinoid preparations. They are thought to trigger specific cannabinoid effects under pharmaceutically controlled conditions. They include CP-55,940, CP-55,244, CP-50,556, CP-47,497, HU-210 (promotes cell growth and has antidepressant effects), HU-211, HU-308, HU-331, RCS-4, RCS-8, rimonabant (= SR-141716A, a selective CB1 inhibitor which was used therapeutically for the treatment of abdominal adiposity), nabilone (see above), 9-nor-9beta-hydroxyhexahydroxycannabinol (= beta-HHC), JWH-015 (triggers cell death, a potential immunosuppressive drug), JWH-018, JWH-019, JWH-051, JWH-073, JWH-081, JWH-122, JWH-133 (inhibits tumor growth), JWH-200, JWH-203, JWH-210, JWH-250, JWH-251, JWH-398, AM-2201, AM-694, AM-411, AM-708, AM-836, AM-855, AM-919, AM-926, AM-938, CB-25, CB-52, WIN 55,212-2, WIN 55,212-3, AMG-1, AMG-3, CT-3 (ajulemic acid), L-759633, 0-1184, 2-isopropyl-5-methyl-1-(2,6-dihydroxy-1,2-dimethylheptylphenyl)cyclohex-1-ene.

Further pharmacologically active cannabinoids known in the art comprise Δ⁸-tetrahydrocannabinol-DMH, Δ⁹-tetrahydrocannabinol propyl analogue (THCV), 11-hydroxy-tetrahydrocannabinol, 11-nor-9-carboxy-tetrahydrocannabinol, 5'-azido-Δ⁸-tetrahydrocannabinol, cannabidiol propyl analogue (cannabidivarin, CBDV), cannabichromene propyl analogue, dimethylheptyl HHC, desacetyl-L-nantradol, dexabinol, levonantradol.

The common denominator of *Cannabis spec*.-derived phytocannabinoids, endocannabinoids and synthetic cannabinoids is that they are extremely lipophilic and consequently poorly water-soluble. Without a suitable formulation they are not really apt for controlled oral administration, either as a pharmaceutically active ingredient or a dietary supplement.

According to the method of the invention the at least one cannabinoid can be solubilized in the overall range of 10 % to 40 % per weight, preferred 20 % to 40 % per weight, more preferred 30 % to 40 % per weight and most preferred 40 % per weight of the first composition, respectively.

Medium-chained triglycerides (MCT) refer to triglycerides whose fatty acids have an aliphatic tail of 6 - 12 carbon atoms. Fatty acids incorporated in MCT are called medium-chain fatty acids (MCFA). In triglycerides three fatty acid molecules are bound to a glycerol backbone. Per definition, in MCT at least two of these three fatty acids must be MCFAs. According to the invention MCFA can be selected independently from one another from the group comprising caproic acid, enanthic acid, caprylic acid, pelargonic acid, capric acid, undecilyc acid, lauric acid, their unsaturated derivatives, and mixtures thereof. Preferred MCFA are caproic acid, caprylic acid, capric acid, and lauric acid.

It can be advantageous in some embodiments of the invention to use triglycerides containing 1 to 3 myristic acid and/or palmitic acid residues instead of MCFAs. Hence, these two fatty acids shall be subsumed under the term MCT according to the invention too.

MCT oils or MCT fats are oils or fats containing predominantly said MCT. These terms refer to a respective mixture of different MCT that may contain a variety of MCFA. According to the invention any reasonable mixing ratio shall be covered by these terms. MCT fats are often extracted from specific plant fats, while MCT oils do not occur naturally. MCT oils and MCT fats are broadly marketed as a healthy dietary supplement, respectively as a surrogate for long-chain fats in nutrition.

According to the method of the invention MCT are used in the overall range of 60 % to 90 % per weight, preferred 60 % to 80 % per weight, more preferred 60 % to 70 % per weight and most preferred 60 % per weight of the first composition, respectively.

Surprisingly, it was found that the preparation of a first composition with the at least one cannabinoid according to the invention and at least one medium-chained triglyceride before joining them with the ingredients of the second composition allows for solubilizing a much higher amount, respectively weight percentage of the at least one cannabinoid according to the invention. Upon joining the first composition and the second composition a final weight percentage of the at least one cannabinoid of up to 36 % can be achieved. In a related method by the Applicant (WO 2018/046120 A1) maximally 25 % per weight can be solubilized. However, such solubilisates having a relative weight percentage over 20 % tend to crystallize after a few days which limits their usability. This is not the case with the solubilisates according to the invention.

The first composition can be either prepared at room temperature (20 ± 5 °C) or it can be pre-heated up to 40 °C. A pre-heating allows for a shorter mixing time with the second composition and hence a shorter time for the temperature ramp in step d) of the method of the invention.

The main ingredient of the second composition according to the invention are non-hydrogenated phosphatidylcholines. These are a class of phospholipids linked to choline. They are a major component of cell membranes and are for example obtained from egg yolk, ox liver, marine animals or soybeans. In practice, it showed that the origin of non-hydrogenated phosphatidylcholines influences their biological and chemical effects considerably. According to the invention the at least one non-hydrogenated phosphatidylcholine (PC) can be selected from the group comprising 1-palmitoyl-2-oleoyl-sn-glycero-3-phosphocholine (POPC), natural (non-hydrogenated) soy bean PC, natural (non-hydrogenated) egg PC, dipalmitoyl phosphatidylcholine (DPPC), dimyristoyl phosphatidylcholine (DMPC) or 1,2-dioleyl-SN-glycero-3-phosphocholine (DOPC), 1-oleoyl-palmitoyl phosphatidylcholine (OPPC), diasteroyl phosphatidylcholine (DSPC), monostearoylphosphatidylcholine (MSPC), diarachidoylphosphatidylcholine (DAPC), corn lecithin, cottonseed oil lecithin, rapeseed lecithin, phosphatidylethanolamine, phosphatidylserine, phosphatidylinositol, phosphatidylinositol polyphosphates, phosphatidylglycerol, phosphatidic acid, phosphatidylinositolamine, diphosphatidylglycerol (cardiolipin), sphingomyelin, ceramide aminoethylphosphonic acid, ceramide phosphorylglycerol, dicetylphosphoric acid, stearylamine, and mixtures thereof. Preferred non-hydrogenated phosphatidylcholines are non-hydrogenated soybean PC, DMPC, POPC and DOPC. Most preferred is non-hydrogenated soybean PC.

Lecithin is commonly used as a synonym for phosphatidylcholines. It is a mixture of phosphatidylcholine and other compounds.

According to the method of the invention non-hydrogenated phosphatidylcholines are used in the overall range of 60 % to 85 % per weight, preferred 70 % to 85 % per weight, more preferred 75 % to 80 % per weight and most preferred 80 % per weight of the second composition, respectively.

Lysophosphatidylcholines (LPC, lysoPC, also: lysolecithins) are a class of derivatives of phosphatidylcholines, resulting of their partial hydrolysis in which one of the fatty acid groups is removed. In the organism this hydrolysis is effected by the enzyme phospholipase A2. According to the invention the at least one non-hydrogenated lysophosphatidylcholine can be selected independently from one another from the group comprising all hydrolyzed compounds of the phosphatidylcholines listed above, 1-lysophosphatidylcholines (2-acyl-sn-glycero-3-phosphocholines), 2-lysophosphatidylcholines, L-alpha-lysophosphatidylcholine, and mixtures thereof.

According to the method of the invention the at least one non-hydrogenated lysophosphatidylcholine is used in the overall range of 5 % to 30 % per weight, preferred 10 % to 20 % per weight, more preferred 12 % to 18 % per weight and most preferred 14 % per weight of the second composition, respectively.

In the scope of the present application said non-hydrogenated lysophosphatidylcholines are not a mere variant or substitute for non-hydrogenated phosphatidylcholines but fulfill an independent role. Surprisingly, it was found that two solubilizing agents of similar but not identical chemical constitution can significantly improve the solubilizing effect, if used in an uneven ratio. According to the invention the ratio non-hydrogenated phosphatidylcholine to non-hydrogenated lysophosphatidylcholine is from 15:1 to 2:1, preferred 12:1 to 4:1, more preferred 10:1 to 6:1 and most preferred 9:1.

According to the invention the at least one C₂ to C₄ alcohol (lower alcohol) can be selected from the group comprising ethanol, propanol, isopropanol, butane-1-ol, butane-2-ol, isobutanol (2-methyl-1-propanol), ethylene glycol (ethane-1,2-diol), α-propylene glycol (propane-1,2-diol), β-propylene glycol (propane-1-3-diol), 1,2-butylene glycol (butane-1,2-diol), 1,3-butylene glycol (butane-1,3-diol), 1,4-butylene glycol (butane-1,4-diol), and diethylene glycol. Preferred is ethanol.

According to the method of the invention C₂ to C₄ alcohols are used in the overall range of 3% to 30 % per weight, preferred 3 % to 20 % per weight, more preferred 3 % to 10 % per weight and most preferred 4 % per weight of the second composition, respectively.

Glyceryl stearate (glycerol monostearate, GMS) is an emulsifier. The flaky powder is also hygroscopic. GMS is used as thickening, emulsifying, anti-caking, anti-staling and preservative agent.

According to the invention the at least one saturated or unsaturated C₁₄ to C₂₀ fatty acid can be used instead of or in combination with glyceryl stearate. It can be selected from the group comprising myristic acid (14:0), pentadecanoic acid (15:0), palmitic acid (16:0), heptadecanoic acid (17:0), stearic acid (18:0), nonadecanoic acid (19:0), arachidic acid (20:0), myristoleic acid (14:1,cis-Δ⁹), palmitoleic acid (16:1, cis-Δ⁹), sapienic acid (16:1, cis-Δ⁶), hexadecatrienoic acid (16:3, (n-3), oleic acid (18:1, cis-Δ⁹), elaidic acid (18:1, trans-Δ⁹), vaccenic acid (18:1, trans-Δ¹¹), linoleic acid (18:2; cis,cis-Δ⁹,Δ¹²), linoleadic acid (18:2, trans, trans-Δ⁹,Δ¹²), α-linolenic acid (18:3, cis,cis,cis-Δ⁹,Δ¹²,Δ¹⁵), γ-linolenic acid (18:3, (*ω*-3)), calendic acid (8E,10E,12Z-octadecatrienoic acid), stearidonic acid (18:4 (n-3)), dihomo-γ-linolenic acid (20:3; (ω-6)), eicosadienoic acid (20:2, (n-6)), eicosatrienoic acid (20:3, (n-3)), eicosatetraenoic acid (20:4, (n-3)), arachidonic acid (20:4, cis,cis,cis,cis-Δ⁵, Δ⁸,Δ¹¹,Δ¹⁴), eicosapentaenoic acid (20:5, cis,cis,cis,cis,cis-Δ⁵, Δ⁸,Δ¹¹,Δ¹⁴,Δ¹⁷). Preferred are even-numbered C₁₄ to C₂₀ fatty acids. Particularly preferred is oleic acid.

According to the method of the invention glyceryl stearate and/or a saturated or unsaturated C₁₄ to C₂₀ fatty acid are used in the overall range of 1 % to 15 % per weight, preferred 1 % to 10 % per weight, more preferred 1% to 5 % per weight and most preferred 1 % per weight.

In Reference Example 4 it was shown that an addition of a cannabinoid to the second composition according to the disclosure instead of to the first composition did not afford a proper solubilization process. The addition of the cannabinoid to the first composition according to the disclosure containing the MCT oil fraction is therefore mandatory.

In Reference Example 5 it was shown that a separation in a first composition and a second composition according to the disclosure is necessary. When all ingredients were added at the same time a precipitate was built in the solubilisate after 3 to 7 days.

When the first composition according to the invention and the second composition according to the invention are mixed the ratio of said first composition to said second composition is in the range of 9:1 to 3:2, preferred 9:1 to 3:1, more preferred 9:1 to 5:1, most preferred 9:1.

In Reference Examples 6 and 7 it was shown that these ratios have an experimental basis. In Reference Example 6 there was a ratio of 20:1 in favor of the first composition. It showed that with an overshoot of the MCT oil phase the solubilization process did not work. In Reference Example 7 the same volumes of the first composition and the second composition according to the disclosure were mixed (ratio 1:1). Under these conditions the mixed solution started to solidify already at room temperature.

It showed that ultrasonic treatment during the mixing step of the first composition and the second composition is advantageous for a good pre-solubilization. Further, it showed that the resulting solubilisates according to the invention are more stable, a clear advantage in respect of pharmaceutical production. The basic principle of ultrasonic treatment is acoustic cavitation. This term refers to the formation of gas- or vapor-filled bubbles in a medium exposed to an oscillating pressure. The passage of an ultrasonic wave will cause oscillations of these bubbles (Suslick et al. (1999) Philos Trans R Soc Lond A 357: 335-353). Surfactants such as phospholipids accumulate at the gas/liquid interface of the cavitating bubble, thereby reducing the surface tension of the bubble which in turn induces an enhanced formation rate of the bubbles (cf. Ashokkumar and Grieser (2007) Phys Chem Chem Phys 9: 5621-5728). The acoustic power needed to induce cavitation is lower at lower frequencies (Mason and Lorimer (1988) Sonochemistry: Theory, Applications, and Uses of Ultrasound in Chemistry. Ellis Horwood, Chichester, N.Y., USA).

There is a broad range of frequencies in sonochemistry that can be used for ultrasonic treatment according to the invention, i.e. 20 kHz - 1 MHz, preferred 20 kHz - 100 kHz, more preferred 20 kHz - 50 kHz, most preferred 20 kHz - 30kHz.

The power that is needed depends mainly on the size of the sample or the batch that is going to be treated with ultrasound. On a laboratory scale, a device with a power of 50 W - 400 W will be useful. For industrial production, however, a device with much larger power is needed, i.e. in a range of 500 W - 40 kW. To reach such a large power several ultrasonic devices can be used at a time to treat a batch. As the method of the disclosure is applicable for small as well as large batches, ultrasound devices over the entire power range are suitable, i.e. from 50 W to 40 kW.

In Reference Example 8 the aforementioned sonification step was left out. The solubilisate did not become completely clear even after the heating step. Upon dissolution of the solubilisate in an aqueous solution so-called zebra striping occurred. The sonification step is thus mandatory for achieving a proper solubilisate according to the disclosure.

An essential feature of the method according to the invention is the temperature control (temperature increment per time and duration of the heating). There is an optimal final temperature for each cannabinoid according to the invention in the range of 100°C to 130°C. The exact value has to be found out empirically.

The continuous temperature increment (the steepness of the temperature ramp) can vary between 0.5°C/min to 5°C/min, preferred 2°C/min to 3°C/min and most preferred 3°C/min.

This heating step serves for a controlled solubilization process. With this heating step the formation of multilamellar vesicles is facilitated in a homogenous self-emulsifying manner when the resulting solubilisate is going to be solubilized in an aqueous solution. This heating step keeps the needed amount of non-hydrogenated phosphatidylcholines and non-hydrogenated lysophosphatidylcholines down and therewith the production costs.

In Reference Example 9 the mixed solution of the first composition and the second composition according to the disclosure underwent ultrasonic treatment and thereupon were heated immediately to over 100°C, instead of running a cautious temperature ramp, as provided in step d) of the method of the disclosure. A solubilization took place but shortly after the cooling down step crystallization of some of the compounds (probably the cannabinoid) started. This temperature ramp is thus an essential step pf the method of the disclosure. As far as Applicant knows, such a cautious temperature ramp is not known in the art for solubilization processes.

In an optional step the solubilisate resulting from the method of the invention can be kept at said final temperature between 100°C and 130°C for one to two hours more. This optional step is particularly useful if a cannabinoid in said solubilisate has been provided at the beginning in its acid form. This is often the case when a *Cannabis* extract is used since the acid form is the predominantly occurring form of many cannabinoids in the plant. In order to be converted into the pharmaceutically far more active form the acid form has to be decarboxylated by heat (cf. Moreno Sanz (2016) Cannabis and Cannabinoid Res 1: 124-130). Examples are tetrahydrocannabinolic acid (THCA) that is converted to Δ⁹-tetrahydrocannabinol (THC) and cannabidiolic acid (CBDA-C₅) that is converted to (-)-cannabidiol (CBD). Upon drying *Cannabis* leaves a minor portion of their cannabinoids is decarboxylated. The major portion is traditionally decarboxylated during smoking by the applied high temperatures, alternatively during baking. This is not the case when one or more cannabinoids shall be administered in an oral form. Therefore, they usually need to be decarboxylated in an extra heating step before processing them. The same holds true if a cannabinoid such as THC or CBD has been isolated and enriched from an extract. The acid form is predominant and needs to be decarboxylated. Depending on the isolation method this includes said extra heating step. With this optional step of the method of the invention this extra heating step has become moot. Thus, the method of the invention facilitates also the providing of a virtually pure active cannabinoid. Each cannabinoid has its own decarboxylation temperature. They are in the range of 100°C to 130°C. Ideally, the final temperature in step d) of the method of the invention is chosen to be said decarboxylation temperature that has only to be maintained in step e) for achieving virtually complete decarboxylation. A complete decarboxylation is normally achieved after one hour at this decarboxylation temperature, maximum after two hours.

Thus, in another embodiment the method of the invention comprises the following steps:
a) Providing a first composition comprising at least one cannabinoid in the overall range of 10 % to 45 % per weight of the first composition and at least one medium-chained triglyceride in the overall range of 55 % to 90 % per weight of the first composition, wherein at least one cannabinoid is provided in its respective acid form, said at least one cannabinoid and said at least one medium-chained triglyceride are mixed under stirring in a temperature range of 15°C to 40°C and the relative weight percentages of the first composition add up to 100%;
b) Providing a second composition comprising at least one non-hydrogenated phosphatidylcholine in the overall range of 60 % to 85 % per weight of the second composition, at least one non-hydrogenated lysophosphatidylcholine in the overall range of 5 % to 30 % per weight of the second composition, at least one C₂-C₄ alcohol in the overall range of 3 % to 30 % per weight of the second composition and at least one of glyceryl stearate or a saturated or unsaturated C₁₄ to C₂₀ fatty acid in the overall range of 1 % to 15 % per weight of the second composition, wherein said ingredients are mixed under stirring at room temperature, the weight ratio of said at least one non-hydrogenated phosphatidylcholine to said one non-hydrogenated lysophosphatidylcholine is in the range of 15:1 to 2:1 and the relative weight percentages of the second composition add up to 100%;
c) Mixing said first composition and said second composition under stirring and the use of ultrasonic treatment in a frequency range of 20 kHz to 1 MHz and a power range of 50 W to 40 KW,
   wherein the weight ratio of said first composition to said second composition is in the range of 9:1 to 3:2;
d) Heating the mixture of step c) by continuously increasing the temperature with a temperature increment of 0.5°C/min to 5°C/min until a final temperature of 100°C to 130°C is reached,
e) Maintaining the final temperature of step d) for a time range of 1 hour to 2 hours; and
f) Letting the resulting solubilisate cool down to room temperature,
wherein no polysorbate or polyethylene glycol is used for the solubilization process.

The methods according to the invention can be performed at a pressure of 0.2 bar to 1 bar. It is preferred, however, to run the method at 1 bar (atmospheric pressure). For certain applications it may be preferable to use a light vacuum. The technical equipment for applying, maintaining and controlling such a light vacuum is well known in the art.

The resulting cannabinoid solubilisate is a clear solution. It does not display any sedimentation, precipitation, slurs, smears or striping (zebra effect).

The cannabinoid solubilisates produced according to the method of the invention maintain this clearness upon cooling down and stay clear and stable upon being stored. The achievable storage time (roughly corresponding to the shelf life of a medicinal product) is apparently not limited. In preliminary stability analyses there was no cannabinoid solubilisate according to the invention where the minimum storage time was less than 14 days. In some cases even a storage time of minimum 12 months could be assessed.

A number of cannabinoids including THC and CBD can undergo oxidation, thus losing or modifying their therapeutic properties (see above). During the solubilization method of the invention an antioxidant can be optionally added (see above). But for augmenting the shelf life of cannabinoid solubilisates containing at least one oxidation-prone cannabinoid it may be advantageous beyond that to add a further antioxidant to the solubilisate when preparing a finished solution. Without the addition of an antioxidant, the afforded solubilisate may unfavorably change in aspect and taste in a relatively short time because of oxidation processes.

In preferred embodiments this at least one further antioxidant is a food additive and/or a pharmaceutically acceptable excipient. Suitable antioxidants can be selected from the group comprising lactic acid, ascorbic acid, sodium ascorbate, calcium ascorbate, potassium ascorbate, fatty acid esters of ascorbic acid, ascorbyl palmitate, ascorbyl stearate, tocopherols, alpha-tocopherol, beta-tocopherol, gamma-tocopherol, delta-tocopherol, alpha-tocotrienol, beta-tocotrienol, gamma-tocotrienol, delta-tocotrienol, propyl gallate, octyl gallate, dodecyl gallate, ethyl gallate, guaiac resin, erythorbic acid, sodium erythorbate, erythorbin acid, sodium erythorbin, tert-butylhydroquinone, butylated hydroxyanisole, butylated hydroxytoluene, mono-, di-, trisodium phosphate, mono-, di-, tripotassium phosphate, anoxomer, ethoxyquin, potassium lactate, stannous chloride, sodium thiosulfate, 4-hexylresorcinol, glucose oxidase. Preferred are ascorbyl palmitate and alpha-tocopherol, beta-tocopherol, gamma-tocopherol, delta-tocopherol. Particularly preferred is a combination of ascorbyl palmitate and at least one of alpha-tocopherol, beta-tocopherol, gamma-tocopherol, delta-tocopherol.

The term tocopherol refers to any of the aforementioned tocopherols or a mixture thereof.

According to the method of the invention this at least one antioxidant can be optionally added to the first composition or the second composition according to the invention in the overall range of 0.01 % to 10 % per weight, preferred 0.1 % to 5 % per weight, more preferred 0.1 % to 1% per weight and most preferred 0.2 % per weight.

Thus, the present application refers also to the solubilisate resulting from the solubilizing method according to the invention:
A solubilisate of at least one cannabinoid, comprising:
a) at least one cannabinoid in the range of 6 % to 40 % per weight;
b) at least one medium-chained triglyceride in the overall range of 33 % to 81 % per weight;
c) at least one non-hydrogenated phosphatidylcholine in the overall range of 6 % to 34 % per weight;
d) at least one non-hydrogenated lysophosphatidylcholine in the overall range of 0.5 % to 15 % per weight;
e) at least one C₂ to C₄ alcohol in the overall range of 0.5 % to 20 % per weight, and
f) at least one of glyceryl stearate or a saturated or unsaturated C₁₄ to C₂₀ fatty acid in the range of 0.5 % to 10 % per weight, respectively,
wherein the solubilisate is free of polysorbates or polyethylene glycols and the relative weight percentages of all ingredients add up to 100 % and all solubilization agents are independently from one another a food additive and/or a pharmaceutically acceptable excipient, and the solubilisate is free of polysorbates or polyethylene glycols.

In a preferred embodiment the solubilisate according to the invention comprises
a) at least one cannabinoid in the range of 40 % per weight;
b) at least one medium-chained triglyceride in the overall range of 50 % per weight;
c) at least one non-hydrogenated phosphatidylcholine in the overall range of 8 % per weight;
d) at least one non-hydrogenated lysophosphatidylcholine in the overall range 1 % per weight;
e) at least one C₂ to C₄ alcohol in the overall range of 0.5 % per weight, and
f) at least one of glyceryl stearate or a saturated or unsaturated C₁₄ to C₂₀ fatty acid in the range of 0.5 % per weight, respectively,
and the solubilisate is free of polysorbates or polyethylene glycols.

According to the invention said solubilisate or its preferred embodiments may additionally contain an antioxidant as listed before in the overall range of 0.01 % to 10 % per weight, preferred 0.1 % to 5 % per weight, more preferred 0.2 % to 1% per weight and most preferred 0.3 % to 0.5 % per weight.

In a particularly preferred embodiment of this solubilisate said at least one C₂ to C₄ alcohol is ethanol.

In a particularly preferred embodiment of this solubilisate said at least one saturated or unsaturated C₁₄ to C₂₀ fatty acid is oleic acid.

In preferred embodiments at least one antioxidant in the overall range of 0.01 % to 6 % per weight is contained additionally in the solubilisate according to the invention, wherein said at least one antioxidant is a food additive and/or a pharmaceutically acceptable excipient.

The term solubility refers to the highest dose strength that is subject to an FDA biowaiver request. Herein, a drug is classified as highly soluble, when the highest dose strength is soluble in 250 ml or less of aqueous media over the pH range of 1 - 7.5. Correspondingly, cannabinoids that cannot be solubilized that way are classified as poorly soluble.

The term permeability refers to the extent of absorption of a drug in humans across the intestinal membrane (mucosa). According to the established definition a drug is classified as highly permeable if 90% or more of the orally administered dose are resorbed in the gastrointestinal tract. Correspondingly, a cannabinoid having an absorption rate of less than 90% is classified as low permeable.

Thus, solubility and permeability are intrinsic substance properties. Resorption and bioavailability, however, describe pharmaceutic parameters that may be improved by suitable measures. While resorption refers to the fraction from the orally applied substance amount that is absorbed from the gastrointestinal tract the bioavailability of a substance depends not only on resorption but also from species-specific protein binding in blood and from pharmacokinetic parameters such as first-pass metabolism.

Thus, the present application refers also to a cannabinoid solubilisate according to the invention for use in a pharmaceutical dosage form, wherein at least one pharmaceutically active cannabinoid is solubilized in said solubilisate.

Moreover, the present application refers also to the use in medicine of the cannabinoid solubilisate according to the invention in a pharmaceutical dosage form.

It is understood that with the method of the invention solubilisates containing at least one cannabinoid can also be produced for use as a dietary supplement. National legislations on specific cannabinoids and possible upper limits of cannabinoid content may apply.

As laid out before, one goal of the solubilisate according to the invention is to enable an augmented resorption and/or bioavailability of the cannabinoid solubilized in said solubilisate. Thus, the present application refers also to a cannabinoid solubilisate according to the invention, in which the solubilisate of the at least one cannabinoid enhances the resorption and/or bioavailability of at least one cannabinoid.

In particular, the present application refers also to a cannabinoid solubilisate produced by the method according to the invention, in which the solubilisate of the at least one cannabinoid enhances the resorption and/or bioavailability of at least one cannabinoid.

A further aspect of the invention is that many cannabinoids have a peculiar smell and/or taste. In *Cannabis* extracts this is attributed to certain terpenes. While this characteristic smell and/or taste is estimated by some people others dislike it decidedly. As terpenes are likewise lipophilic substances, they are co-solubilized when a non-processed cannabinoid extract is going to be solubilized according to the method of the invention. Therefore, a cannabinoid solubilisate according to the invention can significantly help to mask this peculiar smell or taste by caging said terpenes. The smell or taste of cannabidiol extracts is often described as earthy, musky or bitter, that of cannabis oils also as grassy and weedy. In general, cannabinoid taste or smell is often described as bitter. The cannabinoid solubilisates according to the invention use to have a neutral taste, likewise the finished solutions containing a cannabinoid solubilisate according to the invention.

In most cases, the cannabinoid solubilisate itself is not yet a dietary supplement preparation or pharmaceutical dosage form. To be ready for consumption, respectively intake the cannabinoid solubilisate is solved in a diluent. The preferred diluent for oral consumption or oral dosage forms is water. Therefor the solubilisate according to the invention is added to an aqueous solution in a suitable container. The container can be selected from a group comprising, but not limited to, bottles, flasks, vials, flacons, ampoules, glasses, cups, drinking bowls, beverage cartons, Tetra Pak^{®}, cans and custom-built two- or multiple-compartment containers. Preferred containers are bottles and vials.

It is preferred that the container with the aqueous solution and the solubilisate solved therein is shaken several times to ensure a homogeneous distribution of the solubilisate in the aqueous solution and by this way a uniform taste, a constant concentration, avoidance of slurs and an appealing presentation of the finished solution.

Thus, the present application refers also to a finished solution, wherein the cannabinoid solubilisate according to the invention is solved in an aqueous solution.

Moreover, the present application refers also to a pharmaceutical composition containing at least one pharmaceutically active agent formulated in a solubilisate according to the invention or in a finished aqueous solution as defined before, and at least one pharmaceutically acceptable excipient.

The term "pharmaceutical excipients" refers to natural or synthetic compounds that are added to a pharmaceutical formulation alongside the pharmaceutical active agent. They may help to bulk up the formulation, to enhance the desired pharmacokinetic properties or the stability of the formulation, as well as be beneficial in the manufacturing process. Advantageous classes of excipients according to the invention include carriers, binding agents, lubricants, glidants, disintegrants, colorants, buffers, preservatives, emulsifiers, permeation enhancers, antioxidants, diluents, pH-regulators, fatliquors, solvents, consistency enhancers, hydrotropes, sweeteners, acidifiers, thickening agents, anti-adherents, fillers, flavors, sweeteners, opacifiers, flavoring substances and aromatic substances.

It can be advantageous, respectively mandatory to add one or more pharmaceutically acceptable carrier to a pharmaceutically active agent. Eligible are all carriers known in the art and combinations thereof. In solid dosage forms they can be for example plant and animal fats, waxes, paraffins, starch, tragacanth, cellulose derivatives, polyethylene glycols, silicones, bentonites, silica, talcum, zinc oxide. For liquid dosage forms and emulsions suitable carriers are for example solvents, solubilizing agents, emulsifiers such as water, ethanol, isopropanol, ethyl carbonate, ethyl acetate, benzyl alcohol, benzyl benzoate, propylene glycol, 1,3-butyl glycol, cotton seed oil, peanut oil, olive oil, castor oil, sesame oil, glycerol fatty acid esters, polyethyl glycols, fatty acid esters of sorbitan. Suspensions according to the invention may use carriers known in the art such as diluents (e.g. water, ethanol or propylene glycol), ethoxylized isostearyl alcohols, polyoxyethylene and polyoxyethylene sorbitan esters, microcrystalline cellulose, bentonites, agar agar, tragacanth.

The term binding agents refers to substances that bind powders or glue them together, rendering them cohesive through granule formation. They serve as a "glue" of the formulation. Binding agents increase the cohesive strength of the provided diluent or filler.

Suitable binding agents are for example starch from wheat, corn, rice or potato, gelatin, naturally occurring sugars such as glucose, sucrose or beta-lactose, sweeteners from corn, natural and synthetic gums such as acacia, tragacanth or ammonium calcium alginate, sodium alginate, carboxymethyl cellulose, sodium carboxymethyl cellulose, hydroxypropyl carboxymethyl cellulose, polyethylene glycol, polyvinyl pyrrolidone, magnesium aluminum silicate, waxes and others. The percentage of the binding agent in the composition can range from 1 - 30 % by weight, preferred 2 - 20 % by weight, more preferred 3 - 10 % by weight and most preferred 3 - 6 % by weight.

In some embodiments it may be desirable that the prepared beverage generates some foam on being dissolved. Such an effect can be supported through the addition of a foaming agent that reduces the surface tension of the liquid, thus facilitating the formation of bubbles, or it increases its colloidal stability by inhibiting coalescence of bubbles. Alternatively, it may stabilize foam. Suitable examples include mineral oil, quillaia extract, triethyl citrate, sodium lauryl ether sulfate, sodium lauryl sulfate, ammonium lauryl sulfate.

Alternatively, some solubilisates according to the invention may appear slightly foamy upon preparation. Though this does not interfere with the desired application it may affect patient compliance in case of a medication or the commercial success in case of dietary supplements. Therefore, it may be desirable to add a pharmaceutically or nutritionally acceptable anti-foaming agent (defoamer) to the solubilisate. Examples are polydimethylsiloxane or silicone oil in dietary supplements or simethicone in pharmaceuticals.

Colorants are excipients that bestow a colorization to the composition of the drink, respectively the dosage form. These excipients can be food colorants. They can be adsorbed on a suitable adsorption means such as clay or aluminum oxide. The amount of the colorant may vary between 0.01 and 10 % per weight of the composition, preferred between 0.05 and 6 % per weight, more preferred between 0.1 and 4 % per weight, most preferred between 0.1 and 1 % per weight.

Suitable food or pharmaceutical colorants are for example curcumin, riboflavin, riboflavin-5'-phosphate, tartrazine, alkannin, quinolione yellow WS, Fast Yellow AB, riboflavin-5'-sodium phosphate, yellow 2G, Sunset yellow FCF, orange GGN, cochineal, carminic acid, citrus red 2, carmoisine, amaranth, Ponceau 4R, Ponceau SX, Ponceau 6R, erythrosine, red 2G, Allura red AC, Indathrene blue RS, Patent blue V, indigo carmine, Brilliant blue FCF, chlorophylls and chlorophyllins, copper complexes of chlorophylls and chlorophyllins, Green S, Fast Green FCF, Plain caramel, Caustic sulphite caramel, ammonia caramel, sulphite ammonia caramel, Black PN, Carbon black, vegetable carbon, Brown FK, Brown HT, alpha-carotene, beta-carotene, gamma-carotene, annatto, bixin, norbixin, paprika oleoresin, capsanthin, capsorubin, lycopene, beta-apo-8'-carotenal, ethyl ester of beta-apo-8'-carotenic acid, flavoxanthin, lutein, cryptoxanthin, rubixanthin, violaxanthin, rhodoxanthin, canthaxanthin, zeaxanthin, citranaxanthin, astaxanthin, betanin, anthocyanins, saffron, calcium carbonate, titanium dioxide, iron oxides, iron hydroxides, aluminum, silver, gold, pigment rubine, tannin, orcein, ferrous gluconate, ferrous lactate.

Flavor enhancers are widely used for food and drinks. Suitable examples are glutamic acid, monosodium glutamate, monopotassium glutamate, calcium diglutamate, monoammonium glutamate, magnesium diglutamate, guanylic acid, sodium guanylate, disodium guanylate, dipotassium guanylate, calcium guanylate, inosinic acid, disodium inosinate, dipotassium inosinate, calcium inosinate, calcium 5'-ribonucleotides, disodium 5'-ribonucleotides, glycine, sodium glycinate, zinc acetate, gum benzoic, thaumatin, glycyrrhizin, neohesperidine dihydrochalcone, glyceryl monoacetate, glyceryl diacetate.

Moreover, buffer solutions are preferred for liquid formulations, in particular for pharmaceutical liquid formulations. The terms buffer, buffer system and buffer solution, in particular of an aqueous solution, refer to the capacity of the system to resist a pH change by the addition of an acid or a base, or by dilution with a solvent. Preferred buffer systems may be selected from the group comprising formate, lactate, benzoic acid, oxalate, fumarate, aniline, acetate buffer, citrate buffer, glutamate buffer, phosphate buffer, succinate, pyridine, phthalate, histidine, MES (2-(N-morpholino) ethanesulfonic acid, maleic acid, cacodylate (dimethyl arsenate), carbonic acid, ADA (N-(2-acetamido)imino diacetic acid, PIPES (4-piperazine-bis-ethanesulfonic acid), BIS-TRIS propane (1,3-bis[tris(hydroxymethyl)methylamino] propane), ethylene diamine, ACES (2-[(amino-2-oxoethyl)amino]ethanesulfonic acid), imidazole, MOPS (3-(N-morphino)-propanesulfonic acid, diethyl malonic acid, TES (2-[tris(hydroxymethyl)methyl]aminoethanesulfonic acid, HEPES (N-2-hydroxyethylpiperazine-N'-2-ethanesulfonic acid), as well as other buffers with a pKₐ between 3.8 and 7.7.

Preferred are carbonic acid buffers such as acetate buffer and dicarboxylic acid buffers such as fumarate, tartrate and phthalate as well as tricarboxylic acid buffers such as citrate.

A further group of preferred buffers are inorganic buffers such as sulfate hydroxide, borate hydroxide, carbonate hydroxide, oxalate hydroxide, calcium hydroxide and phosphate buffers. Another group of preferred buffers are nitrogen-containing puffers such as imidazole, diethylene diamine and piperazine. Furthermore preferred are sulfonic acid buffers such as TES, HEPES, ACES, PIPES, [(2-hydroxy-1,1-bis-(hydroxymethyl)ethyl)amino]-1-propanesulfonic acid (TAPS), 4-(2-hydroxyethyl)piperazine-1-propanesulfonic acid (EEPS), 4-morpholino-propanesulfonic acid (MOPS) and N,N-bis-(2-hydroxyethyl)-2-aminoethanesulfonic acid (BES). Another group of preferred buffers are glycine, glycyl-glycine, glycyl-glycyl-glycine, *N,N*-bis-(2-hydroxyethyl)glycine and *N*-[2-hydroxy-1,1-bis(hydroxymethyl)ethyl]glycine (tricine). Preferred are also amino acid buffers such as glycine, alanine, valine, leucine, isoleucine, serine, threonine, phenylalanine, tyrosine, tryptophan, lysine, arginine, histidine, aspartate, glutamate, asparagine, glutamine, cysteine, methionine, proline, 4-hydroxy proline, *N,N,N*-trimethyl lysine, 3-methyl histidine, 5-hydroxy-lysine, o-phosphoserine, gamma-carboxyglutamate, [epsilon]-*N*-acetyl lysine, [omega]-*N-*methyl arginine, citrulline, ornithine and their derivatives.

Preservatives for liquid dosage forms or supplements can be used on demand. They may be selected from the group comprising, but not limited to, sorbic acid, potassium sorbate, sodium sorbate, calcium sorbate, methyl paraben, ethyl paraben, methyl ethyl paraben, propyl paraben, benzoic acid, sodium benzoate, potassium benzoate, calcium benzoate, heptyl p-hydroxybenzoate, sodium methyl para-hydroxybenzoate, sodium ethyl para-hydroxybenzoate, sodium propyl para-hydroxybenzoate, benzyl alcohol, benzalkonium chloride, phenylethyl alcohols, cresols, cetylpyridinium chloride, chlorbutanol, thiomersal (sodium 2-(ethylmercurithio) benzoic acid), sulfur dioxide, sodium sulfite, sodium bisulfite, sodium metabisulfite, potassium metabisulfite, potassium sulphite, calcium sulphite, calcium hydrogen sulfite, potassium hydrogen sulfite, biphenyl, orthophenyl phenol, sodium orthophenyl phenol, thiabendazole, nisin, natamycin, formic acid, sodium formate, calcium formate, hexamine, formaldehyde, dimethyl dicarbonate, potassium nitrite, sodium nitrite, sodium nitrate, potassium nitrate, acetic acid, potassium acetate, sodium acetate, sodium diacetate, calcium acetate, ammonium acetate, dehydroacetic acid, sodium dehydroacetate, lactic acid, propionic acid, sodium propionate, calcium propionate, potassium propionate, boric acid, sodium tetraborate, carbon dioxide, malic acid, fumaric acid, lysozyme, copper-(II)-sulfate, chlorine, chlorine dioxide and other suitable substances or compositions known to the person skilled in the art.

Additional emulsifiers can be selected for example from the following anionic and non-ionic emulsifiers: Anionic emulsifier waxes, cetyl alcohol, cetylstearyl alcohol, stearic acid, oleic acid, polyoxyethylene polyoxypropylene block polymers, addition products of 2 to 60 mol ethylene oxide to castor oil and/or hardened castor oil, wool wax oil (lanolin), sorbitan esters, polyoxyethylene alkyl esters, polyoxyethylene sorbitan fatty acid esters, polyoxyethene sorbitan monolaurate, polyoxyethene sorbitan monooleate, polyoxyethene sorbitan monopalmitate, polyoxyethene sorbitan monostearate, polyoxyethene sorbitan tristearate, polyoxyethene stearate, polyvinyl alcohol, metatartaric acid, calcium tartrate, alginic acid, sodium alginate, potassium alginate, ammonium alginate, calcium alginate, propane-1,2-diol alginate, carrageenan, processed eucheuma seaweed, locust bean gum, tragacanth, acacia gum, karaya gum, gellan gum, gum ghatti, glucomannane, pectin, amidated pectin, ammonium phosphatides, brominated vegetable oil, sucrose acetate isobutyrate, glycerol esters of wood rosins, disodium phosphate, trisodium diphosphate, tetrasodium diphosphate, dicalcium diphosphate, calcium dihydrogen diphosphate, sodium triphosphate, pentapotassium triphosphate, sodium polyphosphates, sodium calcium polyphosphate, calcium polyphosphates, ammonium polyphosphate, beta-cyclodextrin, powdered cellulose, methyl cellulose, ethyl cellulose, hydroxypropyl cellulose, hydroxypropyl methylcellulose, ethyl methyl cellulose, carboxymethyl cellulose, sodium carboxymethyl cellulose, ethyl hydroxyethyl cellulose, croscarmellose, enzymically hydrolyzed carboxymethyl cellulose, mono- and diglycerides of fatty acids, glyceryl monostearate, glyceryl distearate, acetic acid esters of mono- and diglycerides of fatty acids, lactic acid esters of mono- and diglycerides of fatty acids, citric acid esters of mono- and diglycerides of fatty acids, tartaric acid esters of mono- and diglycerides of fatty acids, mono- and diacetyl tartaric acid esters of mono- and diglycerides of fatty acids, mixed acetic and tartaric acid esters of mono- and diglycerides of fatty acids, succinylated monoglycerides, sucrose esters of fatty acids, sucroglycerides, polyglycerol esters of fatty acids, polyglycerol polyricinoleate, propane-1,2-diol esters of fatty acids, propylene glycol esters of fatty acids, lactylated fatty acid esters of glycerol and propane-1, thermally oxidized soy bean oil interacted with mono- and diglycerides of fatty acids, dioctyl sodium sulphosuccinate, sodium stearoyl-2-lactylate, calcium stearoyl-2-lactylate, stearyl tartrate, stearyl citrate, sodium stearoyl fumarate, calcium stearoyl fumarate, stearyl tartrate, stearyl citrate, sodium stearoyl fumarate, calcium stearoyl fumarate, sodium laurylsulfate, ethoxylated mono- and diglycerides, methyl glucoside-coconut oil ester, sorbitan monostearate, sorbitan tristrearate, sorbitan monolaurate, sorbitan monooleate, sorbitan monopalmitate, sorbitan trioleate, calcium sodium polyphosphate, calcium polyphosphate, ammonium polyphosphate, cholic acid, choline salts, distarch glycerol, starch sodium octenyl succinate, acetylated oxidized starch.

Preferred are glycerin monooleate and stearic acid.

Stabilizers are substances that can be added to prevent unwanted changes. Though stabilizers are not real emulsifiers they may also contribute to the stability of emulsions, respectively solubilisates. Suitable examples for stabilizers are oxystearin, xanthan gum, agar, oat gum, guar gum, tara gum, polyoxyethene stearate, aspartame-acesulfame salt, amylase, proteases, papain, bromelain, ficin, invertase, polydextrose, polyvinyl pyrrolidone, polyvinyl polypyrrolidone, triethyl citrate, maltitol, maltitol syrup.

Suitable as additional surface-active solubilizing agents (solubilizers) are for example diethylene glycol monoethyl ester, polyethyl propylene glycol co-polymers, cyclodextrins such as α- and β-cyclodextrin, glyceryl monostearates, sorbitan esters, polyoxyethylene glycol, polyoxyethylene sorbitanic acid esters, polyoxyethylene sorbitan monooleate, polyoxyethylene oxystearic acid triglyceride, polyvinyl alcohol, sodium dodecyl sulfate, (anionic) glyceryl monooleates etc.

Suitable aromatic and flavoring substances comprise above all essential oil that can be used for this purpose. In general, this term refers to volatile extracts from plants or parts of plants with the respective characteristic smell. They can be extracted from plants or parts of plants by steam distillation.

Examples are: Essential oils, respectively aromatic substances from sage, cloves, chamomile, anise, star anise, thyme, tea tree, peppermint, mint oil, menthol, cineol, eucalyptus oil, mango, figs, lavender oil, chamomile blossoms, pine needles, cypress, oranges, rosewood, plum, currant, cherry, birch leaves, cinnamon, limes, grapefruit, tangerine, juniper, valerian, lemon balm, lemon grass, palmarosa, cranberry, pomegranate, rosemary, ginger, pineapple, guava, echinacea, ivy leave extract, blueberry, kaki, melons etc. or mixtures thereof, as well as mixtures of menthol, peppermint and star anise oil or menthol and cherry flavor.

These aromatic or flavoring substances can be included in the range of 0.0001 to 10 % per weight (particularly in a composition), preferred 0.001 to 6% per weight, more preferred 0.001 to 4% per weight, most preferred 0.01 to 1% per weight, with regard to the total composition. Application- or single case-related it may be advantageous to use differing quantities.

Suitable sweeteners can be selected from the group comprising, but not limited to, mannitol, glycerol, acesulfame potassium, aspartame, cyclamate, isomalt, isomaltitol, saccharin and its sodium, potassium and calcium salts, sucralose, alitame, thaumatin, glycyrrhizin, neohesperidine dihydrochalcone, steviol glycosides, neotame, aspartame-acesulfame salt, maltitol, maltitol syrup, lactitol, xylitol, erythritol.

Suitable additional solvents may be selected from the group comprising, but not limited to, water, carbonated water, water for injection, water with isotonizing agents, saline, isotonic saline, alcohols, particularly ethyl and n-butyl alcohol, glycols, oleic and linoleic acid triglycerides, caprylic and capric acid mono-, di- and triglycerides, polyoxyethylene caprylic and capric acid glycerides, propylene glycol fatty acid esters, low alkyl fatty acid esters, soy bean oil, propylene glycol laurate, polyoxyethylene glyceryl trioleate, ethyl butyrate, ethyl caprylate, ethyl oleate and mixtures thereof.

Suitable isotonizing agents are for example pharmaceutically acceptable salts, in particular sodium chloride and potassium chloride, sugars such as glucose or lactose, sugar alcohols such as mannitol and sorbitol, citrate, phosphate, borate and mixtures thereof.

Suitable thickening agents can be selected from the group comprising, but not limited to, polyvinyl pyrrolidone, methyl cellulose, hydroxypropyl methyl cellulose, hydroxypropyl cellulose, dextrins, polydextrose, modified starch, alkaline modified starch, bleached starch, oxidized starch, enzyme-treated starch, monostarch phosphate, distarch phosphate esterified with sodium trimetaphosphate or phosphorus oxychloride, phosphate distarch phosphate, acetylated distarch phosphate, starch acetate esterified with acetic anhydride, starch acetate esterified with vinyl acetate, acetylated distarch adipate, acetylated distarch glycerol, distarch glycerin, hydroxypropyl starch, hydroxy propyl distarch glycerin, hydroxypropyl distarch phosphate, hydroxypropyl distarch glycerol, starch sodium octenyl succinate, acetylated oxidized starch, hydroxyethyl cellulose.

Diluents or fillers are inactive substances added to drugs in order to handle minimal amounts of active agents. They can be useful in the solubilizing process. Examples for suitable diluents are water, mannitol, pre-gelatinized starch, starch, microcrystalline cellulose, powdered cellulose, silicified microcrystalline cellulose, dibasic calcium phosphate dihydrate, calcium phosphate, calcium carbonate, hydroxypropyl cellulose, hydroxyethyl cellulose, hydroxypropyl methylcellulose, polyethylene glycol, xanthum gum, gum arabic or any combination thereof.

Opacifiers are substances that render the drinkable liquid opaque, if desired. They must have a refractive index substantially different from the solvent, in most cases here water. At the same time, they should be inert to the other components of the composition. Suitable examples include titanium dioxide, talc, calcium carbonate, behenic acid, cetyl alcohol, or mixtures thereof.

According to the invention all of the aforementioned excipients and classes of excipients can be used without limitation alone or in any conceivable combination thereof, as long as the inventive use of a solubilisate is not thwarted, toxic actions may occur or the respective national legislations are infracted.

Thus, the present application refers also to a pharmaceutical composition according to the invention for use in medicine.

The aforementioned solubilisates of at least one cannabinoid, when used as a dietary supplement, can be combined with a variety of additives, as laid out in the following:
Suitable vitamins are for example vitamin C (L-ascorbic acid, sodium L-ascorbate, calcium L-ascorbate, potassium L-ascorbate, L-ascorbyl 6-palmitate), vitamin A (retinol, retinyl acetate, retinyl palmitate, beta-carotene), vitamin D (cholealciferol, ergoalciferol), vitamin E (D-alpha-tocopherol, DL-alpha-tocopherol, D-alpha-tocopheryl acetate, DL-alpha-tocopheryl acetate, D-alpha-tocopheryl succinate), vitamin K (phylloquinone), vitamin B1 (thiamin hydrochloride, thiamin mononitrate), vitamin B2 (riboflavin, sodium riboflavin 5'-phosphate), niacin (nicotinic acid, nicotinamide), pantothenic acid (calcium D-pantothenate, sodium D-pantothenate, D-panthenol), vitamin B6 (pyridoxine hydrochloride, pyridoxine 5'-phosphate), folic acid (pteroyl monoglutaminic acid), vitamin B12 (cyanocobalamine, hydroxocobalamine), biotin (D-biotin).

Suitable minerals to be included are for example calcium (calcium carbonate, calcium chloride, citric acid calcium salt, calcium gluconate, calcium glycerophosphate, calcium lactate, ortho-phosphoric acid calcium salt, calcium hydroxide, calcium oxide), magnesium (magnesium acetate, magnesium carbonate, magnesium chloride, citric acid magnesium salt, magnesium gluconate, magnesium glycerophosphate, ortho-phosphoric acid magnesium salt, magnesium lactate, magnesium hydroxide, magnesium oxide, magnesium sulfate), iron (iron carbonate, iron citrate, iron ammonium citrate, iron gluconate, iron fumarate, iron sodium diphosphate, iron lactate, iron sulfate, iron diphosphate, ferric saccharate, elemental iron), copper (copper carbonate, copper citrate, copper gluconate, copper sulfate, copper lysine complex), iodine (sodium iodide, sodium iodate, potassium iodide, potassium iodate), zinc (zinc acetate, zinc chloride, zinc citrate, zinc gluconate, zinc lactate, zinc oxide, zinc carbonate, zinc sulfate), manganese (manganese carbonate, manganese chloride, manganese citrate, manganese gluconate, manganese glycerophosphate, manganese sulfate), sodium (sodium bicarbonate, sodium carbonate, sodium chloride, sodium citrate, sodium gluconate, sodium lactate, sodium hydroxide, ortho-phosphoric acid sodium salt), potassium (potassium bicarbonate, potassium carbonate, potassium chloride, potassium citrate, potassium gluconate, potassium glycerophosphate, potassium lactate, potassium hydroxide, ortho-phosphoric acid potassium salt), selenium (sodium selenite, sodium hydrogen selenite, sodium selenite), chrome (chrome-(III)-chloride, chrome-(III)-sulfate), molybdenum (ammonium molybdate (molybdenum (VI), sodium molybdate (molybdenum (VI)), fluorine (sodium fluoride, potassium fluoride), chlorine, phosphor.

Trace elements are dietary minerals that are needed by the organism in very small amounts for growth, development and physiology, for example as co-enzymes. Some of them are virtually always present in the organism in sufficient quantities, others have to be substituted in persons in need thereof. They can be selected from the group comprising, but not limited to, chrome, cobalt, iron, iodine, copper, manganese, molybdenum, selenium, zinc, fluoride, silicon, arsenic, nickel, rubidium, tin, vanadium. They can be substituted either as a pure element or in any of the mineral forms mentioned above.

Stimulants are often and worldwide used in drinks. According to the World Health Organization (WHO) this term refers to any kind of substances increasing, accelerating or improving neuronal activity. These substances have often a psychomimetic effect. Most popular stimulants include xanthines such as caffeine, theophylline and theobromine. Guaraná contains the aforementioned xanthines. A further popular stimulant is nicotine, respectively nicotinic acid. However, there is a broad group of stimulants that in many countries are banned by law, expected to be banned in the near future, or underlie a strict regulation of health authorities, needing the prescription of a physician. This is due to their dependence potential and other hazards to consumers' health, attention deficits in traffic etc., or negative effects on social life. This group includes a.o. amphetamine and its derivatives, a group of piperazine derivatives, cocaine and drugs for the treatment of narcolepsy and attention deficit hyperactivity disorder (ADHD). Hence the use of this group of substances according to the invention may be possible, but is discouraged, if legally banned. Preferred is the use of caffeine.

Further suitable antioxidants can be selected from the group comprising lactic acid, ascorbic acid, sodium ascorbate, calcium ascorbate, potassium ascorbate, fatty acid esters of ascorbic acid, ascorbyl palmitate, ascorbyl stearate, tocopherols, alpha-tocopherol, gamma-tocopherol, delta-tocopherol, propyl gallate, octyl gallate, dodecyl gallate, ethyl gallate, guaiac resin, erythorbic acid, sodium erythorbate, erythorbin acid, sodium erythorbin, tert-butylhydroquinone, butylated hydroxyanisol, butylated hydroxytoluene, mono-, di-, trisodium phosphate, mono-, di-, tripotassium phosphate, anoxomer, ethoxyquin, potassium lactate, stannous chloride, sodium thiosulfate, 4-hexylresorcinol, glucose oxidase.

Suitable acidity regulators can be selected from the group comprising acetic acid, potassium acetate, sodium acetate, sodium diacetate, calcium acetate, carbon dioxide, malic acid, fumaric acid, sodium lactate, potassium lactate, calcium lactate, ammonium lactate, magnesium lactate, citric acid, mono-, di-, trisodium citrate, mono-, di-, tripotassium citrate, mono-, di-, tricalcium citrate, tartaric acid, mono-, disodium tartrate, mono-, dipotassium tartrate, sodium potassium tartrate, ortho-phosphoric acid, lecithin citrate, magnesium citrate, ammonium malate, sodium malate, sodium hydrogen malate, calcium malate, calcium hydrogen malate, adipic acid, sodium adipate, potassium adipate, ammonium adipate, succinic acid, sodium fumarate, potassium fumarate, calcium fumarate, ammonium fumarate, 1,4-heptonolactone, triammonium citrate, ammonium ferric citrate, calcium glycerophosphate, isopropyl citrate, potassium carbonate, potassium bicarbonate, ammonium carbonate, ammonium bicarbonate, magnesium carbonate, magnesium bicarbonate, ferrous carbonate, ammonium sulfate, aluminum potassium sulfate, aluminum ammonium sulfate, sodium hydroxide, potassium hydroxide, ammonium hydroxide, magnesium hydroxide, gluconic acid.

Acidifiers use to be inorganic chemicals that either produce or become acid. Suitable examples are: Ammonium chloride, calcium chloride.

### EXAMPLES

In the ensuing examples the relative quantities of the solubilizing agents can be changed inside the margins indicated for each component in the method according to the invention. The addition of tocopherol is optional.

The resulting solubilisate can be diluted with an aqueous solution in order to obtain a finished solution. The volume of the aqueous solution can vary according to the desired volume of the finished solution.

It is possible to upscale or downscale the indicated amounts according to the desired absolute amount of the agent to be solubilized in the solubilisate. The solubilisate can be portioned according to the desired final amount of the agent that shall be administered to a patient in need thereof.

In general, the afforded solubilisates produced according to the method of the invention had a specific gravity of 0.92 - 0.94.

Standard chemicals were purchased from Sigma-Aldrich, Darmstadt, Germany.

### Example 1: Solubilization of Δ⁹-tetrahydrocannabinol

Ca. 100 ml of a solubilisate of Δ⁹-tetrahydrocannabinol were generated by the following procedure:
40 g Δ⁹-tetrahydrocannabinol (Ashanoa, Austria; purity > 98 %) were mixed with 50 ml MCT oil (Azelis, Moers, Germany) under stirring for 10 min at room temperature and atmospheric pressure to generate a first composition.

8 g 1-palmitoyl-2-oleoyl-sn-glycero-3-phosphocholine (POPC; Lipoid GmbH, Ludwigshafen, Germany),), 1 g L-alpha-lysophosphatidylcholine (Lipoid GmbH, Ludwigshafen, Germany), 500 µl ethanol, 300 µl oleic acid and 200 mg glyceryl stearate were mixed under stirring for 5 min at room temperature and atmospheric pressure to generate a second composition.

The first composition and the second composition were mixed under stirring for 10 min at room temperature and atmospheric pressure. During the mixing ultrasound was applied to the joint composition via a BANDELIN SONOPULS HD 2070.2 (generator: GM 2070.2; ultrasonic converter: UW 2070; stepped standard horn SH 70 G; microtip MS 73, 3 mm; BANDELIN electronics GmbH & Co. KG, Berlin, Germany) at 100 W and 20 kHz.

Then the joint composition was heated under continuous stirring with an approximate temperature increment of 4°C / min. Thus, a clear solubilisate according to the invention was obtained after ca. 20 min at a final temperature of ca. 100°C. Then the heating and the stirring was stopped and the resulting solubilisate was allowed to cool down to room temperature. The solubilisate stayed clear and stable over min. 1 month.

Upon being diluted into an aqueous finished solution (10 ml solubilisate; bidest. water ad 100 ml) under stirring the finished solution became quickly clear and had an ivory to light brownish appearance. The taste of the finished solution was neutral.

### Example 2: Solubilization of (-)-cannabidiol

Ca. 100 ml of a solubilisate of (-)-cannabidiol were generated by the following procedure:
40 g (-)-cannabidiol (Ashanoa, Austria; purity > 98 %) were mixed with 50 ml MCT oil (Azelis, Moers, Germany) under stirring for 10 min at room temperature and atmospheric pressure to generate a first composition.

7 g dimyristoyl phosphatidylcholine (DMPC; Lipoid GmbH, Ludwigshafen, Germany), 1.5 g of a mixture of 1-lysophosphatidylcholine and 2-lysophosphatidylcholine (weight ratio: 1:1; Lipoid GmbH, Ludwigshafen, Germany), 700 µl ethanol, 400 µl oleic acid, 200 mg glyceryl stearate and 200 mg alpha-tocopherol were mixed under stirring for 5 min at room temperature and atmospheric pressure to generate a second composition.

The first composition and the second composition were mixed under stirring for 10 min at room temperature and atmospheric pressure. During the mixing ultrasound was applied to the joint composition via a BANDELIN SONOPULS HD 2070.2 (generator: GM 2070.2; ultrasonic converter: UW 2070; stepped standard horn SH 70 G; microtip MS 73, 3 mm; BANDELIN electronics GmbH & Co. KG, Berlin, Germany) at 100 W and 20 kHz.

Then the joint composition was heated under continuous stirring with an approximate temperature increment of 5°C / min. Thus, a clear solubilisate according to the invention was obtained after ca. 20 min at a final temperature of ca. 120°C. Then the heating and the stirring was stopped and the resulting solubilisate was allowed to cool down to room temperature. The solubilisate stayed clear and stable over min. 6 weeks.

Upon being diluted into an aqueous finished solution (10 ml solubilisate; bidest. water ad 100 ml) under stirring the finished solution became quickly clear and had an ivory to light brownish appearance. The taste of the finished solution was neutral. The unpleasant taste of cannabidiol was completely covered.

### Example 3: Solubilization of a Cannabis sativa extract

Ca. 100 ml of a solubilisate of a *Cannabis sativa* extract were generated by the following procedure:
40 g *Cannabis sativa* extract (Ashanoa, Austria) were mixed with 50 ml MCT oil (Azelis, Moers, Germany) under stirring for 15 min at room temperature and atmospheric pressure to generate a first composition.

8 g non-hydrogenated soy bean PC and POPC (weight ratio: 1:1; Lipoid GmbH, Ludwigshafen, Germany), 1 g L-alpha-lysophosphatidylcholine (Lipoid GmbH, Ludwigshafen, Germany), 500 µl ethanol, 300 µl oleic acid and 200 mg ascorbyl palmitate were mixed under stirring for 5 min at room temperature and atmospheric pressure to generate a second composition.

The first composition and the second composition were mixed under stirring for 15 min at room temperature and atmospheric pressure. During the mixing ultrasound was applied to the joint composition via a BANDELIN SONOPULS HD 2070.2 (generator: GM 2070.2; ultrasonic converter: UW 2070; stepped standard horn SH 70 G; microtip MS 73, 3 mm; BANDELIN electronics GmbH & Co. KG, Berlin, Germany) at 100 W and 20 kHz.

Then the joint composition was heated under continuous stirring with an approximate temperature increment of 3°C / min. Thus, a clear solubilisate according to the invention was obtained after ca. 33 min at a final temperature of ca. 120°C. Then the heating and the stirring was stopped and the resulting solubilisate was allowed to cool down to room temperature. The solubilisate stayed clear and stable over min. one month.

Upon being diluted into an aqueous finished solution (10 ml solubilisate; bidest. water ad 100 ml) under stirring the finished solution became quickly clear and had an ivory to light brownish appearance. The taste of the finished solution was neutral. The grassy taste of *Cannabis* extracts was completely covered.

### Reference Example 4: Solubilization of (-)-cannabidiol: Variation I

The experiment was conducted with the same ingredients and relative percentages as Example 2. The difference was that (-)-cannabidiol was not included in the first but in the second composition (the phosphatidylcholine composition).

The result was that (-)-cannabidiol was not solved properly in the phosphatidylcholine phase. Upon joining the first composition and the second composition, as provided in step c) of the process of the disclosure, the solubilization process did not properly work. Neither temperature increase nor sonification led to a clear solubilisate. This approach was not further pursued.

### Reference Example 5: Solubilization of (-)-cannabidiol: Variation II

The experiment was conducted with the same ingredients and relative percentages as Example 2. The difference was that that there was no separation in a first and a second composition but all ingredients were added in one step and the method of the disclosure starts with step c).

The result was that the solubilization process took more time (> 30 minutes) until the solution became clear. The temperature increase, however, was likewise stopped at 120°C. A problem showed with the stability. After 3 - 7 days substances from the solubilisate started to build a precipitate at the bottom of the vial. Simple agitation could not solve the precipitate again. As long-term stability is needed for a commercial product this approach was not further pursued.

### Reference Example 6: Solubilization of (-)-cannabidiol: Variation III

The experiment was conducted with the same ingredients and relative percentages as Example 2. The difference was that the mixing ratio of the first composition to the second composition in step c) of the method of the disclosure was 20:1.

The result was that the solubilization process according to the disclosure did not work. The solubilization process according to the disclosure did not work. Neither temperature increase nor sonification led to a clear solubilisate.

### Reference Example 7: Solubilization of (-)-cannabidiol: Variation IV

The experiment was conducted with the same ingredients and relative percentages as Example 2. The difference was that the mixing ratio of the first composition to the second composition in step c) of the method of the disclosure was 1:1.

The result was that the solubilization process according to the disclosure did not work. The mixture started to solidify at room temperature. Neither temperature increase nor sonification led to a clear solubilisate.

### Reference Example 8: Solubilization of (-)-cannabidiol: Variation V

The experiment was conducted with the same ingredients and relative percentages as Example 2. The difference was that no ultrasonic treatment was used for the mixture of the first composition and the second composition, as provided in step c) of the method of the disclosure.

The result was that the awarded solubilisate did not become completely clear. Moreover, upon solving the solubilisate in an aqueous solution so called zebra-striping occurred. The sonification step seems therefore necessary for the method of the invention.

### Reference Example 9: Solubilization of (-)-cannabidiol: Variation VI

The experiment was conducted with the same ingredients and relative percentages as Example 2. The difference was that the mixture of the first composition and the second composition of step c) of the method of the disclosure was not cautiously heated, as provided in step d) of the method of the disclosure, but was heated immediately to over 100°C.

The result was that the solubilization process according to the disclosure yielded a clear solution. But shortly after cooling down a crystallization process started. Crystals (apparently of (-)-cannabidiol) started to precipitate in a considerable degree. The cautious heating step seems therefore necessary for the method of the invention.

## Claims

1. A method for solubilizing a cannabinoid, comprising the following steps:
a) Providing a first composition comprising at least one cannabinoid in the overall range of 10 % to 45 % per weight of the first composition and at least one medium-chained triglyceride in the overall range of 55 % to 90 % per weight of the first composition, wherein said at least one cannabinoid and said at least one medium-chained triglyceride are mixed under stirring in a temperature range of 15°C to 40°C and the relative weight percentages of the first composition add up to 100%;
b) Providing a second composition comprising at least one non-hydrogenated phosphatidylcholine in the overall range of 60 % to 85 % per weight of the second composition, at least one non-hydrogenated lysophosphatidylcholine in the overall range of 5 % to 30 % per weight of the second composition, at least one C₂-C₄ alcohol in the overall range of 3 % to 30 % per weight of the second composition and at least one of glyceryl stearate or a saturated or unsaturated C₁₄ to C₂₀ fatty acid in the overall range of 1 % to 15 % per weight of the second composition, wherein said ingredients are mixed under stirring at room temperature, the weight ratio of said at least one non-hydrogenated phosphatidylcholine to said one non-hydrogenated lysophosphatidylcholine is in the range of 15:1 to 2:1 and the relative weight percentages of the second composition add up to 100%;
c) Mixing said first composition and said second composition under stirring and the use of ultrasonic treatment in a frequency range of 20 kHz to 1 MHz and a power range of 50 W to 40 KW,
wherein the weight ratio of said first composition to said second composition is in the range of 9:1 to 3:2;
d) Heating the mixture of step c) by continuously increasing the temperature with a temperature increment of 0.5°C/min to 5°C/min until a final temperature of 100°C to 130°C is reached,
e) Optionally maintaining the final temperature of step d) for a time range of 1 hour to 2 hours; and
f) Letting the resulting solubilisate cool down to room temperature,
wherein no polysorbate or polyethylene glycol is used for the solubilization process.

2. The method according to claim 1, wherein said at least one saturated or unsaturated C₁₄ to C₂₀ fatty acid is oleic acid.

3. The method according to claim 1 or 2, wherein said at least one C₂ to C₄ alcohol is ethanol.

4. The method according to any one of claims 1 to 3, wherein additionally in step b) at least one antioxidant in the overall range of 0.01 % to 10 % per weight is added, said at least one antioxidant being a food additive and/or a pharmaceutically acceptable excipient.

5. The method according to claim 4, wherein said at least one antioxidant is ascorbyl palmitate and/or at least one tocopherol.

6. A solubilisate of at least one cannabinoid, produced by a method as defined in any one of claims 1 to 5.

7. The solubilisate as defined in claim 6 for use in medicine.

8. The solubilisate for use according to claim 7 for masking the smell or taste of the at cannabinoid.

9. The solubilisate according to claim 7, in which the solubilisate of the at least one cannabinoid enhances the resorption or bioavailability of at least one cannabinoid.

10. The solubilisate according to claims 6 or 9, in which the at least one cannabinoid is (-)-cannabidiol.

11. A finished solution, wherein a solubilisate as defined in claim 6 is solved in an aqueous solution.

12. A pharmaceutical composition containing at least one cannabinoid formulated in a solubilisate as defined in claim 6 or in a finished solution as defined in claim 11 and at least one pharmaceutically acceptable excipient, respectively.

13. A pharmaceutical composition according to claim 12, wherein said at least one pharmaceutically acceptable excipient is selected from a group comprising carriers, binding agents, lubricants, glidants, disintegrants, colorants, buffers, preservatives, emulsifiers, permeation enhancers, antioxidants, diluents, pH-regulators, fatliquors, solvents, consistency enhancers, hydrotropes, sweeteners, acidifiers, thickening agents, anti-adherents, fillers, flavors, sweeteners, opacifiers, flavoring substances and aromatic substances.

14. A pharmaceutical composition according to claim 12 or 13 for use in medicine.

15. A pharmaceutical composition according to any one of claims 12 to 14, wherein the at least one cannabinoid is Δ⁹-tetrahydrocannabinol or (-)-cannabidiol.

## Patentansprüche

1. Ein Verfahren zur Solubilisierung eines Cannabinoids, das die folgenden Schritte umfasst:
a) Bereitstellen einer ersten Zusammensetzung, die mindestens ein Cannabinoid im Gesamtbereich von 10 bis 45 Gew.-% der ersten Zusammensetzung und mindestens ein mittelkettiges Triglycerid im Gesamtbereich von 55 bis 90 Gew.-% der ersten Zusammensetzung umfasst, wobei das mindestens eine Cannabinoid und das mindestens eine mittelkettige Triglycerid unter Rühren in einem Temperaturbereich von 15 bis 40 °C gemischt werden und die relativen Gewichtsprozente der ersten Zusammensetzung sich zu 100 % addieren;
b) Bereitstellen einer zweiten Zusammensetzung, die mindestens ein nicht hydriertes Phosphatidylcholin in einem Gesamtbereich von 60 bis 85 Gew.-% der zweiten Zusammensetzung, mindestens ein nicht hydriertes Lysophosphatidylcholin in einem Gesamtbereich von 5 bis 30 Gew.-% der zweiten Zusammensetzung, mindestens einen C₂-C₄-Alkohol im Gesamtbereich von 3 bis 30 Gew.-% der zweiten Zusammensetzung und mindestens eines von Glycerylstearat oder einer gesättigten oder ungesättigten C₁₄- bis C₂₀-Fettsäure im Gesamtbereich von 1 bis 15 Gew.-% der zweiten Zusammensetzung umfasst, wobei die Bestandteile unter Rühren bei Raumtemperatur gemischt werden, das Gewichtsverhältnis des mindestens einen nicht hydrierten Phosphatidylcholins zu dem einen nicht hydrierten Lysophosphatidylcholin im Bereich von 15:1 bis 2:1 und die relativen Gewichtsprozente der zweiten Zusammensetzung sich zu 100 % summieren;
c) Mischen der ersten Zusammensetzung und der zweiten Zusammensetzung unter Rühren und Anwendung von Ultraschallbehandlung in einem Frequenzbereich von 20 kHz bis 1 MHz und einem Leistungsbereich von 50 W bis 40 KW,
wobei das Gewichtsverhältnis der ersten Zusammensetzung zur zweiten Zusammensetzung im Bereich von 9:1 bis 3:2 liegt;
d) Erhitzen der Mischung aus Schritt c) durch kontinuierliche Erhöhung der Temperatur mit einem Temperaturinkrement von 0,5°C/min bis 5°C/min, bis eine Endtemperatur von 100°C bis 130°C erreicht ist;
e) Optional Aufrechterhalten der Endtemperatur aus Schritt d) über einen Zeitraum von 1 Stunde bis 2 Stunden; und
f) Abkühlen lassen des resultierenden Solubilisates auf Raumtemperatur,
wobei kein Polysorbat oder Polyethylenglykol bei dem Solubilisierungsprozess verwendet wird.

2. Das Verfahren gemäß Anspruch 1, wobei die mindestens eine gesättigte oder ungesättigte C₁₄- bis C₂₀-Fettsäure Ölsäure ist.

3. Das Verfahren gemäß Anspruch 1 oder 2, wobei der mindestens eine C₂- bis C₄-Alkohol Ethanol ist.

4. Das Verfahren gemäß einem der Ansprüche 1 bis 3, wobei in Schritt b) zusätzlich mindestens ein Antioxidans im Gesamtbereich von 0,01 bis 10 Gew.-% zugesetzt wird, wobei das mindestens eine Antioxidans ein Lebensmittelzusatzstoff und/oder ein pharmazeutisch verträglicher Hilfsstoff ist.

5. Das Verfahren gemäß Anspruch 4, wobei das mindestens eine Antioxidans Ascorbylpalmitat und/oder mindestens ein Tocopherol ist.

6. Ein Solubilisat von mindestens einem Cannabinoid, hergestellt nach einem Verfahren gemäß einem der Ansprüche 1 bis 5.

7. Das Solubilisat gemäß Anspruch 6 zur Verwendung in der Medizin.

8. Das Solubilisat zur Verwendung gemäß Anspruch 7 zur Maskierung des Geruchs oder Geschmacks des mindestens einen Cannabinoids.

9. Das Solubilisat gemäß Anspruch 7, wobei das Solubilisat des mindestens einen Cannabinoids die Resorption oder Bioverfügbarkeit des mindestens einen Cannabinoids erhöht.

10. Das Solubilisat gemäß Anspruch 6 oder 9, bei dem das mindestens eine Cannabinoid (-)-Cannabidiol ist.

11. Eine fertige Lösung, wobei ein Solubilisat gemäß Anspruch 6 in einer wässrigen Lösung gelöst ist.

12. Eine pharmazeutische Zusammensetzung, die mindestens ein Cannabinoid, das in einem Solubilisat gemäß Anspruch 6 oder in einer fertigen Lösung gemäß Anspruch 11 formuliert ist, und mindestens einen pharmazeutisch verträglichen Hilfsstoff enthält.

13. Eine pharmazeutische Zusammensetzung gemäß Anspruch 12, wobei der mindestens eine pharmazeutisch verträgliche Hilfsstoff aus einer Gruppe ausgewählt ist, die Trägerstoffe, Bindemittel, Schmiermittel, Gleitmittel, Sprengmittel, Farbstoffe, Puffer, Konservierungsmittel, Emulgatoren, Permeationsverstärker, Antioxidantien, Verdünnungsmittel, pH-Regulatoren, Fettungsmittel, Lösungsmittel, Konsistenzverstärker, Befeuchtungsmittel, Süßstoffe, Säuerungsmittel, Verdickungsmittel, Antihaftmittel, Füllstoffe, Geschmacksstoffe, Süßstoffe, Trübungsmittel, Aromastoffe und aromatische Substanzen umfasst.

14. Eine pharmazeutische Zusammensetzung gemäß Anspruch 12 oder 13 zur Verwendung in der Medizin.

15. Eine pharmazeutische Zusammensetzung gemäß einem der Ansprüche 12 bis 14, wobei das mindestens eine Cannabinoid Δ⁹-Tetrahydrocannabinol oder (-)-Cannabidiol ist.

## Revendications

1. Un procédé de solubilisation d'un cannabinoïde, comprenant les étapes suivantes :
a) Fournir une première composition comprenant au moins un cannabinoïde dans la gamme totale de 10 à 45 % en poids de la première composition et au moins un triglycéride à chaîne moyenne dans la gamme totale de 55 à 90 % en poids de la première composition, dans laquelle le au moins un cannabinoïde et le au moins un triglycéride à chaîne moyenne sont mélangés sous agitation dans une gamme de température de 15 à 40°C et les pourcentages en poids relatifs de la première composition s'ajoutent pour donner 100 %;
b) Fournir une seconde composition comprenant au moins une phosphatidylcholine non hydrogénée dans une gamme totale de 60 à 85 % en poids de la seconde composition, au moins une lysophosphatidylcholine non hydrogénée dans une gamme totale de 5 à 30 % en poids de la seconde composition, au moins un alcool en C₂ à C₄ dans une gamme totale de 3 à 30 % en poids de la seconde composition et au moins un de stéarate glycéride. % de la seconde composition et au moins un stéarate de glycéryle ou un acide gras saturé ou insaturé en C₁₄ à C₂₀ dans la gamme totale de 1 à 15 % en poids de la seconde composition, les ingrédients étant mélangés sous agitation à température ambiante, le rapport en poids de ladite au moins une phosphatidylcholine non hydrogénée à ladite au moins une lysophosphatidylcholine non hydrogénée étant dans la gamme de 15:1 à 2:1, et les pourcentages en poids relatifs de la seconde composition s'ajoutent pour donner 100 % ;
c) Mélanger la première composition et la seconde composition tout en agitant et en appliquant un traitement ultrasonique dans une gamme de fréquences de 20 kHz à 1 MHz et une gamme de puissances de 50 W à 40 KW,
le rapport en poids de la première composition à la seconde composition étant dans la gamme de 9:1 à 3:2 ;
d) Chauffer le mélange de l'étape c) en augmentant continuellement la température avec un incrément de température de 0,5°C/min à 5°C/min jusqu'à ce qu'une température finale de 100°C à 130°C soit atteinte ;
e) Optionnellement, maintenir la température finale de l'étape d) pendant une période de 1 heure à 2 heures ; et
f) Laisser refroidir le solubilisât résultant jusqu'à la température ambiante,
**caractérisé en ce qu'**aucun polysorbate ou polyéthylène glycol ne soit utilisé dans le procédé de solubilisation.

2. Le procédé selon la revendication 1, dans lequel ledit au moins un acide gras saturé ou insaturé en C₁₄ à C₂₀ est l'acide oléique.

3. Le procédé selon la revendication 1 ou 2, dans lequel ledit au moins un alcool en C₂ à C₄ est l'éthanol.

4. Le procédé selon l'une quelconque des revendications 1 à 3, dans lequel, à l'étape b), au moins un antioxydant est ajouté en outre dans la gamme totale de 0,01 à 10 % en poids, ledit au moins un antioxydant étant un additif alimentaire et/ou un excipient pharmaceutiquement acceptable.

5. Le procédé selon la revendication 4, dans lequel ledit au moins un antioxydant est le palmitate d'ascorbyle et/ou au moins un tocophérol.

6. Un solubilisât d'au moins un cannabinoïde produit par un procédé selon l'une quelconque des revendications 1 à 5.

7. Le solubilisât selon la revendication 6 pour l'utilisation en médecine.

8. Le solubilisât pour l'utilisation selon la revendication 7 pour masquer l'odeur ou la saveur dudit au moins un cannabinoïde.

9. Le solubilisât selon la revendication 7, dans lequel le solubilisât dudit au moins un cannabinoïde augmente l'absorption ou la biodisponibilité dudit au moins un cannabinoïde.

10. Le solubilisât selon la revendication 6 ou 9, dans lequel le au moins un cannabinoïde est le (-)-cannabidiol.

11. Une solution finale, dans laquelle un solubilisât selon la revendication 6 est dissous dans une solution aqueuse.

12. Une composition pharmaceutique comprenant au moins un cannabinoïde formulé dans un solubilisât selon la revendication 6 ou dans une solution finale selon la revendication 11, et au moins un excipient pharmaceutiquement acceptable.

13. Une composition pharmaceutique selon la revendication 12, dans laquelle ledit au moins un excipient pharmaceutiquement acceptable est choisi dans un groupe comprenant des véhicules, liants, glissants, lubrifiants, désintégrants, colorants, tampons, conservateurs, émulsifiants, amplificateurs de perméation, antioxydants, diluants, régulateurs de pH, graissants, solvants, renforceurs de consistance, hydrotropes, édulcorants, acidifiants, épaississants, antiadhésifs, charges, arômes, édulcorants, opacifiants, arômes et substances aromatiques.

14. Une composition pharmaceutique selon la revendication 12 ou 13 pour l'utilisation en médecine.

15. Une composition pharmaceutique selon l'une quelconque des revendications 12 à 14, dans laquelle ledit au moins un cannabinoïde est le Δ-9-tétrahydrocannabinol ou le (-)-cannabidiol.
